Europäisches Patentamt

European Patent Office

Office européen des brevets

(19)

(11) **EP 1 165 673 B1**

(12) ## EUROPÄISCHE PATENTSCHRIFT

(45) Veröffentlichungstag und Bekanntmachung des Hinweises auf die Patenterteilung:
**18.12.2002 Patentblatt 2002/51**

(51) Int Cl.$^7$: **C08J 9/30**, C08J 9/12,
A61L 15/60, A61L 15/42

(21) Anmeldenummer: **00907592.0**

(22) Anmeldetag: **21.02.2000**

(86) Internationale Anmeldenummer:
**PCT/EP00/01407**

(87) Internationale Veröffentlichungsnummer:
**WO 00/052087 (08.09.2000 Gazette 2000/36)**

(54) **WASSERABSORBIERENDE, SCHAUMFÖRMIGE, VERNETZTE POLYMERISATE MIT VERBESSERTER VERTEILUNGSWIRKUNG, VERFAHREN ZU IHRER HERSTELLUNG UND IHRE VERWENDUNG**

WATER-ABSORBING, CELLULAR, CROSS-LINKED POLYMERS WITH IMPROVED DISTRIBUTION EFFECT, METHOD FOR THEIR PRODUCTION AND THEIR USE

POLYMERES HYDROPHILES RETICULES SOUS FORME DE MOUSSE A EFFET DE DISPERSION AMELIORE, PROCEDE DE FABRICATION ET D'UTILISATION DESDITS POLYMERES

(84) Benannte Vertragsstaaten:
**DE ES FR GB IT SE**

(30) Priorität: **03.03.1999 DE 19909214**

(43) Veröffentlichungstag der Anmeldung:
**02.01.2002 Patentblatt 2002/01**

(73) Patentinhaber: **BASF AKTIENGESELLSCHAFT**
**67056 Ludwigshafen (DE)**

(72) Erfinder:
• **HÄHNLE, Hans-Joachim**
**D-67435 Neustadt (DE)**
• **SCHRÖDER, Ulrich**
**D-67227 Frankenthal (DE)**
• **HEIDER, Wolfgang**
**D- 67434 Neustadt (DE)**
• **SCHORNICK, Gunnar**
**D-67271 Neuleiningen (DE)**

• **ANSTOCK, Thomas**
**D-67256 Weisenheim (DE)**

(56) Entgegenhaltungen:
**WO-A-97/17397          WO-A-99/44648**
**US-A- 5 712 316**

• **HARADA ET AL: "Manufacture of water-absorbing sheets with easy handling and excellent flexibility and water absorption properties and their water-absorbing products" , STN CHEMICAL ABSTRAC XP002110926 & JP 08 073507 A (NIPPON SHOKUBAI CO LTD) 19. März 1996 (1996-03-19)**
• **DATABASE WPI Section Ch, Week 199350 Derwent Publications Ltd., London, GB; Class A14, AN 1993-402042 XP002137842 & KR 9 303 797 B (LUCKY C), 13. Mai 1993 (1993-05-13)**

**Beschreibung**

Die Erfindung betrifft wasserabsorbierende, schaumförmige, vernetzte Polymerisate mit verbesserter Verteilungswirkung, Verfahren zur ihrer Herstellung und ihre Verwendung in Sanitärartikeln, die zur Absorption von Körperflüssigkeiten und in Verbandmaterial zur Abdeckung von Wunden eingesetzt werden.

Wasserabsorbierende, vernetzte Polymerisate werden als Superabsorber oder superabsorbierende Polymere bezeichnet, weil sie in der Lage sind, ein Vielfaches ihres Eigengewichtes an wäßrigen Flüssigkeiten unter Ausbildung von Hydrogelen aufzunehmen. In der Praxis werden Superabsorber beispielsweise in Windeln zur Absorption von Urin eingesetzt. Die Superabsorber haben die Eigenschaft, die absorbierte Flüssigkeit auch unter mechanischer Belastung zurückzuhalten. Sie können z.B. als Granulat oder als Pulver sowie in geschäumter Form vorliegen. Man kennt zwei unterschiedliche Typen von Schäumen, (1) Mischungen, die Superabsorber in einer geschäumten Matrix enthalten, und (2) Schäume, die aus einem superabsorbierenden Material bestehen.

Ein unter die Kategorie (1) fallender Schaum wird beispielsweise aus einer Mischung hergestellt, die einerseits Komponenten für die Bildung eines Polyurethanschaums und andererseits polymerisierbare Monomere, einen Vernetzer und einen Polymerisationsinitiator zur Herstellung eines Superabsorbers enthält. Aus einer solchen Mischung wird in einer Polykondensationsreaktion aus den Polyurethankomponenten der Schaum gebildet, der den durch Polymerisation der Monomeren entstehenden Superabsorber in Form eines interpenetrierenden Netzwerkes enthält, vgl. US-A-4 725 628, US-A-4 725 629 und US-A-4 731 391.

Aus der US-A-4 985 467 ist ein Polyurethanschaum bekannt, der einen Superabsorber chemisch gebunden enthält, während in WO94/07935, WO95/32860, WO96/16099, WO96/31555 und WO98/14508 Polyurethanschäume beschrieben werden, die Partikeln von superabsorbierenden Polymeren (SAP) ohne chemische Anbindung enthalten. Außerdem sind Kombinationen von Latex-Schäumen bekannt, in die nach dem Schäumprozeß superabsorbierende, feinteilige Materialien eingearbeitet werden, vgl. EP-A-427 219 und US-A-4 990 541. In US 5506277 wird ein geschlossenzelliger Stärke-Schaum beschrieben, der 20-40 % Superabsorber enthält.

Schäume diesen Typs besitzen den grundsätzlichen Nachteil, daß der in der Schaummatrix enthaltene Superabsorber während der Absorption von wässrigen Flüssigkeiten das Porenvolumen ausfüllt und so die Porenstruktur des Schaums immer weiter blockiert. Daher weisen diese Materialien eine mit zunehmender Absorption deutlich abfallende Aufnahmegeschwindigkeit und eine immer schlechter werdende Verteilungswirkung auf. Dieses Verhalten ist für den Einsatz in Hygieneartikeln sehr nachteilig.

Zu der Kategorie (2) von Schäumen gehören beispielsweise solche Produkte, die dadurch erhalten werden, daß man einen vorgefertigten Superabsorber in einem Extruder mit einer Polyhydroxyverbindung und einem Treibmittel bei erhöhter Temperatur mischt. Beim Auspressen der Mischung aus dem Extruder bildet sich der Schaum. Verfahren dieser Art sind beispielsweise in der US-A-4 394 930, US-A-4 415 388 und US 4410571 beschrieben.

Aus US-A-4 529 739 und US-A-4 649 164 sind Verfahren zur Herstellung von Schäumen bekannt, wobei man ein wasserquellbares, COOH-Gruppen tragendes Polymer mit einem Treibmittel aufschäumt, das in einer Neutralisierungsreaktion mit den COOH-Gruppen des Polymeren das Treibgas freisetzt.

Aus der WO-A-88/09801 ist bekannt, daß man hydrophile Polymere, z.B. Polynatriumacrylat in Gegenwart von Vernetzern wie Polyepoxiden und Treibmitteln durch Erwärmen zu einem schaumförmigen Superabsorber verarbeiten kann.

Gemäß den Angaben in der WO-A-94/22502 werden superabsorbierende Schäume auf Basis von vernetzten, teilweise neutralisierten Polycarboxylaten dadurch hergestellt, daß man eine Monomermischung mit einem in Wasser unlöslichen Treibmittel schäumt, das einen Siedepunkt unterhalb von $50°C$ hat, und den Schaum praktisch gleichzeitig mit dem Schäumen auspolymerisiert. Nachteilig bei diesem Verfahren ist zum einen der Einsatz großer Treibmittelmengen insbesondere der Einsatz von FCKW und zum anderen die aufwendige Kontrolle der Prozeßführung.

Aus der EP-A-0421264 ist die Herstellung schaumartiger Superabsorber bekannt, wobei man eine wäßrige Monomermischung, die eine Ölphase emulgiert enthält, polymerisiert. Das Öl wirkt hierbei als Platzhalter für die späteren Poren des Schaums und wird nach beendeter Polymerisation beim Trocknen des schaumförmigen Materials durch Verdampfen entfernt.

Zur Herstellung schaumförmiger Superabsorber ist außerdem eine Arbeitsweise bekannt, bei der man Carbonate, Hydrogencarbonate oder Kohlendioxid als Treibmittel zu einer Mischung aus Carboxylgruppen tragenden Monomeren, Vernetzungsmittel und Polymerisationsinitiator zusetzt, wobei zeitgleich mit der Zugabe des Treibmittels oder kurz darauf die Polymerisation der Monomeren gestartet wird. Der Superabsorber erhält durch das bei der Neutralisationsreaktion gebildete Kohlendioxid eine Schaumstruktur, vgl. DE-A 3831261, US-A 5118719, EP-A-538983, US-A-4 808 637 und US-A-5750585. Nach dem aus WO-A-95/02002 und EP-A-644207 bekannten Verfahren wird ein geschäumter und gemahlener Superabsorber im Anschluß an die Herstellung mit einer oder mehreren zur nachträglichen Oberflächenvernetzung reaktionsfähigen Verbindungen versetzt und auf eine Temperatur von 100 bis $300°C$ erhitzt.

Die Vorgehensweise, den Schäumungsprozeß und die Polymerisation weitgehend synchron ablaufen zu lassen, bedingt eine Reihe von Nachteilen. So werden häufig Schäume mit inhomogenen Schaumstrukturen erhalten, die z.T. erhebliche Anteile an geschlossenzelligen Strukturen enthalten. Fernerhin ist eine starke Tendenz zur Hautbildung zu beobachten und die erhaltenen Materialien sind hart und spröde. Diese Probleme haben zur Konsequenz, daß diese Schäume nicht als ganze Schaumkörper sondern nur in gemahlener Form Anwendung finden, wie in den vorstehend genannten Veröffentlichungen ausgeführt ist. Durch den Mahlprozeß wird die geschilderte Inhomogenität beseitigt. Außerdem werden dabei die geschlossenzelligen Strukturen bzw. die Hautschichten des geschäumten Materials aufgebrochen.

In der JP-A-08073507 werden weiche und flexible Superabsorberfilme schrieben, die dadurch herstellt werden, daß eine wäßrige Acrylatlösung, die zum Teil durch ein Alkanolamin neutralisiert ist, in Gegenwart eines Vernetzungsmittels polymerisiert wird. Die erhaltenen Filme werden zwar als weich und flexibel beschrieben, aber ihre anwendungstechnischen Eigenschaften sind völlig unzureichend. So ist ihre Wasseraufnahmegeschwindigkeit für die Anwendung in Hygieneartikeln viel zu gering, außerdem zeigen sie keinerlei Verteilungswirkung und weisen eine die Handhabung stark einschränkende, ausgeprägte Klebrigkeit auf.

Aus der DE-A-19607551 sind wasserabsorbierende, schaumförmige, vernetzte Polymerisate bekannt, die erhältlich sind durch

    (I) Schäumen einer polymerisierbaren wäßrigen Mischung, die

        (a) Säuregruppen enthaltende monoethylenisch ungesättigte Monomere, die zu mindestens 50 Mol-% neutralisiert sind,

        (b) gegebenenfalls andere monoethylenisch ungesättigte Monomere.

        (c) Vernetzer,

        (d) Initiatoren,

        (e) 0,1 bis 20 Gew.-% mindestens eines Tensids,

        (f) gegebenenfalls mindestens einen Lösevermittler und

        (g) gegebenenfalls Verdicker, Schaumstabilisatoren, Polymerisationsregler, Füllstoffe und/oder Zellkeimbildner enthält, wobei das Schäumen durch Dispergieren von feinen Blasen eines gegenüber Radikalen inerten Gases erfolgt, und

    (II) Polymerisieren der geschäumten Mischung unter Bildung eines schaumförmigen Hydrogels und gegebenenfalls Einstellen des Wassergehalts des Polymerisats auf 1 bis 60 Gew.-%.

Gegenstand der WO-A-97/17397 sind wasserabsorbierende, schaumförmige, vernetzte Polymerisate, die erhältlich sind durch (I) Schäumen einer polymerisierbaren wäßrigen Mischung, die (a) Säuregruppen enthaltende monoethylenisch ungesättigte Monomere, die zu mindestens 50 Mol-% neutralisiert sind; (b) gegebenenfalls andere monoethylenisch ungesättigte Monomere; (c) Vernetzer; (d) Initiatoren; (e) 0,1 bis 20 Gew.-% mindestens eines Tensids; (f) gegebenenfalls mindestens einen Lösevermittler und (g) gegebenenfalls Verdicker, Schaumstabilisatoren, Polymerisationsregler, Füllstoffe und/oder Zellkeimbildner enthält, wobei das Schäumen durch Dispergieren von feinen Blasen eines gegenüber Radikalen inerten Gases erfolgt; und (II) Polymerisieren der geschäumten Mischung unter Bildung eines schaumförmigen Hydrogels und Einstellen des Wassergehalts des schaumförmigen Polymerisats auf 1 bis 45 Gew.-%. Die so erhältlichen schaumförmigen Polymerisate werden in Sanitärartikeln verwendet, die zur Absorption von Körperflüssigkeiten eingesetzt werden.

Aus der nicht vorveröffentlichten WO-A-99/44648 sind wasserabsorbierende, schaumförmige, vernetzte Polymerisate bekannt, die erhältlich sind durch (I) Schäumen einer polymerisierbaren wässrigen Mischung, die (a) Säuregruppen enthaltende monoethylenisch ungesättigte Monomere, die gegebenenfalls neutralisiert sind, (b) gegebenenfalls andere monoethylenisch ungesättigte Monomere, (c) Vernetzer, (d) Initiatoren, (e) 0,1 bis 20 Gew.-% mindestens eines Tensids, (f) gegebenenfalls mindestens einen Lösevermittler und (g) gegebenenfalls Verdicker, Schaumstabilisatoren, Polymerisationsregler, Füllstoffe und/oder Zellkeimbildner enthält, wobei das Schäumen durch Dispergieren von feinen Blasen eines gegenüber Radikalen inerten Gases in die polymerisierbare wässrige Mischung erfolgt, und (II) Polymerisieren der geschäumten Mischung unter Bildung eines schaumförmigen Hydrogels, wobei mindestens 20 Mol-% der Säuregruppen enthaltenden Monomeren (a) mit tertiären Alkanolaminen neutralisiert und/oder die freies Säuregruppen des schaumförmigen Hydrogels nach dem Polymerisieren zu mindestens 20 Mol-% mit mindestens einem Alkanolamin neutralisiert worden sind. Die schaumförmigen Polymerisate werden ebenfalls in Sanitärartikeln, die zur Absorption von Körperflüssigkeiten eingesetzt werden und in Verbandmaterial zur Abdekkung von Wunden verwendet.

Ein wesentliches Problem bei der Herstellung von superabsorbierenden Schäumen aus Monomermischungen stellt die Temperaturkontrolle dar. Wie in WO-A-94/22502 und in US-A-5750585 ausführlich erläutert, kommt der Temperaturkontrolle während des Aufschäumungs-und Polymerisationsprozesses eine entscheidende Bedeutung zu. Nur bei strikter Kontrolle des Temperaturverlaufs ist die Erzielung der gewünschten offenzelligen Schaumstrukturen gewährleistet. Auch im Falle des Verfahrens der DE-A-19607551 ist die Temperaturkontrolle während des Aufschlagsvorgangs von wesentlicher Bedeutung, weil sie die Struktur des erzeugten Monomerschaums und damit des Polymerschaums entscheidend mitbeinflußt.

Eine strikte Temperaturkontrolle ist im technischen Maßstab immer dann problematisch und sehr aufwendig zu realisieren, wenn gut isolierende Produkte wie Schäume vorliegen und wenn bei dem Prozeß gleichzeitig große Wärmemengen freigesetzt werden. Große Wärmemengen werden einerseits durch die enormen Polymerisationswärmen der eingesetzten Monomeren geliefert andererseits durch den hohen Energieeintrag, der im Falle des Verfahrens der DE-A-19607551 bei der Schlagschaumherstellung auftritt.

Ein grundsätzliches Problem für die Anwendung von Superabsorbern in der Hygieneindustrie ist deren mangelnde Fähigkeit zur Verteilung von Flüssigkeiten. Dieser Mangel ist insbesondere bei pulverförmigen Superabsorbern zu beobachten, aber auch die vorstehend aufgelisteten Schäume erreichen nicht das gewünschte Eigenschaftsniveau. Diesem Problem wird gerade in jüngster Zeit verstärkt Aufmerksamkeit gewidmet, weil aufgrund des Trends zu immer dünneren Hygieneartikeln der Anteil des Cellulosefluffs, das bisher die Verteilung übernommen hat, immer weiter erniedrigt wird. Es besteht somit der Wunsch nach superabsorbierenden Materialien, die nicht nur eine gute Speicherwirkung und eine rasche Aufnahmecharakteristik, sondern vor allem eine ausgeprägte Veteilungswirkung aufweisen.

Aufgabe der vorliegenden Erfindung ist es einen superabsorbierenden Schaum zur Verfügung zu stellen, der gegenüber den bekannten, vergleichbaren Schäumen eine deutlich verbesserte Verteilungswirkung aufweist.

Die Aufgabe wird erfindungsgemäß gelöst mit wasserabsorbierenden, schaumförmigen, vernetzten Polymerisaten, die eine freie Aufnahmegeschwindigkeit (FAG) für eine 0,9 gew.-%ige wäßrige Kochsalzlösung von 4,0 bis 100 g/g sec. haben und die erhältlich sind durch

(I) Schäumen einer polymerisierbaren wäßrigen Mischung, die

(a) Säuregruppen enthaltende monoethylenisch ungesättigte Monomere oder mit Alkalimetallbasen, Ammoniak oder Aminen neutralisierte Säuregruppen enthaltende monoethylenisch ungesättigte Monomere,
(c) Vernetzer,
(d) Initiatoren und
(e) 0,1 bis 20 Gew.-% mindestens eines Tensids,

enthält, wobei man zum Schäumen ein gegenüber Radikalen inertes Gas unter einem Druck von 2 bis 400 bar in der polymerisierbaren wäßrigen Mischung löst und sie anschließend auf Atmosphärendruck entspannt und
(II) Polymerisieren der geschäumten Mischung unter Bildung eines schaumförmigen Hydrogels.

Bevorzugt sind solche wasserabsorbierenden, schaumförmigen, vernetzten Polymerisate, die erhältlich sind durch Neutralisation der Säuregruppen enthaltenden Monomeren (a) mit tertiären Alkanolaminen und/oder durch Neutralisation der freien Säuregruppen des schaumförmigen Hydrogels nach dem Polymerisieren mit mindestens einem Alkanolamin, wobei der Neutralisationsgrad jeweils mindestens 20 Mol.-% und vorzugsweise mindestens 40 Mol.-% beträgt.

Gegenstand der Erfindung ist außerdem ein Verfahren zur Herstellung von wasserabsorbierenden, schaumförmigen, vernetzten Polymerisaten durch Schäumen einer polymerisierbaren Mischung aus

(a) Säuregruppen enthaltenden monoethylenisch ungesättigten Monomeren oder mit Alkalimetallbasen, Ammoniak oder Aminen neutralisierte Säuregruppen enthaltende monoethylenisch ungesättigte Monomere,
(c) Vernetzer,
(d) Initiatoren und
(e) 0,1 bis 20 Gew.-% mindestens eines Tensids

in einer ersten Verfahrensstufe mit einem gegenüber Radikalen inertem Gas und Polymerisieren des so enthaltenen Schaums in einer zweiten Verfahrensstufe unter Bildung eines schaumförmigen Hydrogels, wobei man in der ersten Verfahrensstufe ein gegenüber Radikalen inertes Gas unter einem Druck von 2 bis 400 bar in der polymerisierbaren wäßrigen Mischung löst und sie anschließend unter Schaumbildung auf Atmosphärendruck entspannt.

Durch diese Vorgehensweise gelingt es, superabsorbierende Schäume in reproduzierbarer Weise herzustellen, die im Vergleich zu herkömmlichen Schäumen aus SAP neben einer verbesserten Verteilungswirkung auch noch eine schnellere Aufnahmecharakteristik aufweisen und die in reproduzierbarer Weise homogene, offenzellige Strukturen ohne Tendenz zur Hautbildung besitzen. Fernerhin entfällt das Problem der Temperaturkontrolle während der Herstellung weitgehend. Die Monomerschaumerzeugung verläuft nahezu ohne wärmetönung, so daß die Temperaturkontrolle einfach durch die anfänglich eingestellte Temperatur gewährleistet ist. Die stark exotherme Polymerisation kann adiabatisch geführt werden, ohne daß dadurch wesentliche Einbußen in den Produkteigenschaften in Kauf genommen werden müßten. Ein weiterer Vorteil dieses Verfahrens besteht darin, daß die Reaktionsmischungen sehr hohe Feststoffgehalte aufweisen können (bis zu 90%), wodurch der Trocknungsaufwand erheblich reduziert werden kann.

Eine eventuell auftretende Klebrigkeit der geschäumten Materialien kann durch eine zusätzliche Puderung mit feinteiligen, hydrophilen Pulvern gelöst werden.

Erfindungsgemäß wird eine polymerisierbare wäßrige Mischung zu einem Schaum verarbeitet, der verarbeitungsstabil ist und beliebig geformt werden kann. Die polymerisierbare wäßrige Mischung enthält als Komponenten (a) Säuregruppen enthaltende monoethylenisch ungesättigte Monomere, die gegebenenfalls neutralisiert sind. Solche Monomere sind beispielsweise monoethylenisch ungesättigte $C_3$- bis $C_{25}$-Carbonsäuren oder Anhydride, beispielsweise Acrylsäure, Methacrylsäure, Ethacrylsäure, $\alpha$-Chlpracrylsäure, Crotonsäure, Maleinsäure, Maleinsäureanhydrid, Itaconsäure, Citraconsäure, Mesaconsäure, Glutaconsäure, Aconitsäure und Fumarsäure.

Als Monomere der Gruppe (a) kommen außerdem monoethylenisch ungesättigte Sulfonsäuren in Betracht, beispielsweise Vinylsulfonsäure, Allylsulfonsäure, Sulfoethylacrylat, Sulfoethylmethacrylat, Sulfopropylacrylat, Sulfopropylmethacrylat, 2-Hydroxy-3-acryloxypropylsulfonsäure, 2-Hydroxy-3-methacryloxypropylsulfonsäure, Vinylphosphonsäure, Allylphosphonsäure und 2-Acrylamido-2-methylpropansulfonsäure. Die Monomeren können allein oder in Mischung untereinander bei der Herstellung der superabsorbierenden Schäume eingesetzt werden. Bevorzugt eingesetzte Monomere der Gruppe (a) sind Acrylsäure, Methacrylsäure, Vinylsulfonsäure, Acrylamidopropansulfonsäure oder Mischungen dieser Säuren, z.B. Mischungen aus Acrylsäure und Methacrylsäure, Mischungen aus Acrylsäure und Acrylamidopropansulfonsäure oder Mischungen aus Acrylsäure und Vinylsulfonsäure.

Die Monomeren sind gegebenenfalls neutralisiert. Zur Neutralisation verwendet man beispielsweise Alkalimetallbasen oder Ammoniak bzw. Amine. Zur Neutralisation setzt man vorzugsweise Natronlauge oder Kalilauge ein. Die Neutralisation kann jedoch auch mit Hilfe von Natriumcarbonat, Natriumhydrogencarbonat, Kaliumcarbonat oder Kaliumhydrogencarbonat oder anderen Carbonaten, Hydrogencarbonaten oder Ammoniak vorgenommen werden. Die Säuregruppen der Monomeren werden beispielsweise zu 15 bis 90 Mol-% mit mindestens einer der oben angegebenen Basen neutralisiert.

Bei einer Ausführungsform des erfindungsgemäßen Verfahrens neutralisiert man die Monomeren (a) zu mindestens 20 Mol-% mit tertiären Alkanolaminen. Besonders bevorzugt ist eine Ausführungsform, bei der man mindestens 40 Mol-% der Säuregruppen enthaltenden Monomeren (a) mit tertiären Alkanolaminen neutralisiert. Hierbei kann man die Monomeren (a) gegebenenfalls zusätzlich mit den oben beschriebenen Basen, insbesondere NaOH oder Ammoniak, bis zu beispielsweise 100 % neutralisieren. Der Neutralisationsgrad der Säuregruppen enthaltenden Monomeren (a) mit tertiären Alkanolaminen beträgt bei dieser Verfahrensvariante 20 bis 95, vorzugsweise 30 bis 70 Mol-%. Bevorzugt verwendete tertiäre Amine sind Triethanolamin, Methyldiethanolamin, Dimethylaminodiglykol, Dimethylethanolamin und N,N,N' ,N'-Tetra-(hydroxyethyl)ethylendiamin.

Die polymerisierbare wäßrige Mischung kann gegebenenfalls Monomere der Gruppe (b) enthalten. Hierunter sollen andere monoethylenisch ungesättigte Monomere verstanden werden, die mit den Monomeren (a) und (c) copolymerisierbar sind. Hierzu gehören beispielsweise die Amide und Nitrile von monoethylenisch ungesättigten Carbonsäuren, z.B. Acrylamid, Methacrylamid und N-Vinylformamid, Acrylnitril und Methacrylnitril, Dialkyldiallylammoniumhalogenide wie Dimethyldiallylammoniumchlorid, Diethyldiallylammoniumchlorid, Allylpiperidiniumbromid, N-Vinylimidazole wie z.B. N-Vinylimidazol, 1-Vinyl-2-methylimidazol und N-Vinylimidazoline wie N-Vinylimidazolin, 1-Vinyl-2-methylimidazolin, 1-Vinyl-2-ethylimidazolin oder 1-Vinyl-2-propylimidazolin, die in Form der freien Basen, in quaternisierter Form oder als Salz bei der Polymerisation eingesetzt werden können. Außerdem eignen sich Dialkylaminoalkylacrylate und Dialkylaminoalkylmethacrylate, Dimethylaminoethylacrylat, Dimethylaminoethylmethacrylat, Diethylaminoethylacrylat und Diethylaminoethylmethacrylat. Die basischen Ester werden vorzugsweise in quaternisierter Form oder als Salz eingesetzt. Weitere geeignete Verbindungen der Gruppe (b) sind beispielsweise Vinylester von gesättigten $C_1$- bis $C_4$-Carbonsäuren wie Vinylformiat, Vinylacetat oder Vinylpropionat, Alkylvinylether mit mindestens 2 C-Atomen in der Alkylgruppe, wie z.B. Ethylvinylether oder Butylvinylether, Ester von monoethylenisch ungesättigten $C_3$- bis $C_6$-Carbonsäuren, z.B. Ester aus einwertigen $C_1$- bis $C_{18}$-Alkoholen und Acrylsäure, Methacrylsäure oder Maleinsäure, Halbester von Maleinsäure, z.B. Maleinsäuremonomethylester. Andere geeignete Monomere der Gruppe (b) sind Styrol, alkylsubstituierte Styrole wie Ethylstyrol oder tert. Butylstyrol.

Die Monomeren der Gruppe (b) können auch in Mischung untereinander bei der Copolymerisation mit den Monomeren (a) und (c) eingesetzt werden, z.B. Mischungen aus Vinylacetat und Ethylacrylat in beliebigem Verhältnis.

Die Monomeren der Gruppe (c) haben mindestens 2 ethylenisch ungesättigte Doppelbindungen. Beispiele für solche Monomere, die bei Polymerisationsreaktionen üblicherweise als Vernetzer eingesetzt werden, sind N,N'-Methylen-bisacrylamid, Polyethylenglykoldiacrylate und Polyethylenglykoldimethacrylate, die sich jeweils von Polyethylenglykolen eines Molekulargewichts von 106 bis 8500, vorzugsweise 400 bis 2000, ableiten, Trimethylolpropantriacrylat, Trimethylolpropantrimethacrylat, Ethylenglykoldiacrylat, Propylenglykoldiacrylat, Butandioldiacrylat, Hexandioldiacrylat, Hexandioldimethacrylat, Allylmethacrylat, Diacrylate und Dimethacrylate von Blockcopolymerisaten aus Ethylenoxid und Propylenoxid, zweifach bzw. dreifach mit Acrylsäure oder Methacrylsäure veresterte mehrwertige Alkohole, wie Glycerin oder Pentaerythrit, Triallylamin, Tetraallylethylendiamin, Divinylbenzol, Diallylphthalat, Polyethylenglykoldivinylether von Polyethylenglykolen eines Molekulargewichts von 106 bis 4000, Trimethylolpropandiallylether, Butandioldivinylether, Pentaerythrittriallylether und/oder Divinylethylenharnstoff. Vorzugsweise setzt man wasserlösliche Vernetzer ein, z.B. N,N'-Methylen-bisacrylamid, Polyethylenglykoldiacrylate und Polyethylenglykoldimethacrylate, die sich von Additionsprodukten von 2 bis 400 Mol Ethylenoxid an 1 Mol eines Diols oder Polyols ableiten, Vinylether von Additionsprodukten von 2 bis 400 Mol Ethylenoxid an 1 Mol eines Diols oder Polyols, Ethylenglykoldiacrylat, Ethylenglykoldimethacrylat oder Triacrylate und Trimethacrylate von Additionsprodukten von 6 bis 20 Mol Ethylenoxid an ein Mol Glycerin, Pentaerythrittriallylether und/oder Divinylharnstoff.

Als Vernetzer kommen außerdem Verbindungen in Betracht, die mindestens eine polymerisierbare ethylenisch ungesättigte Gruppe und mindestens eine weitere funktionelle Gruppe enthalten. Die funktionelle Gruppe dieser Vernetzer muß in der Lage sein, mit den funktionellen Gruppen, im wesentlichen den Carboxylgruppen oder Sulfonsäuregruppen der Monomeren (a) zu reagieren. Geeignete funktionelle Gruppen sind beispielsweise Hydroxyl-, Amino-, Epoxi- und Aziridinogruppen.

Als Vernetzer kommen außerdem solche Verbindungen in Betracht, die mindestens zwei funktionelle Gruppen tragen, die mit Carboxyl- und Sulfonsäuregruppen der eingesetzten Monomeren der Gruppe (a) reagieren können. Die geeigneten funktionellen Gruppen wurden bereits oben genannt, d.h. Hydroxyl-, Amino-, Epoxi-, Isocyanat-, Ester-, Amide- und Aziridinogruppen. Beispiele für solche Vernetzer sind Ethylenglykol, Diethylenglykol, Triethylenglykol, Tetraethylenglykol, Polyethylenglykol, Glycerin, Polyglycerin, Propylenglykol, Polypropylenglykol, Blockcopolymerisate aus Ethylenoxid und Propylenoxid, Sorbitanfettsäureester, ethoxylierte Sorbitanfettsäureester, Trimethylolpropan, Pentaerythrit, 1,3-Butandiol, 1,4-Butandiol, Polyvinylalkohol, Sorbit, Polyglycidylether wie Ethylenglykoldiglycidylether, Polyethylenglykoldiglycidylether, Glycerindiglycidylether, Glycerinpolyglycidylether, Diglycerinpolyglycidylether, Polyglycerinpolyglycidylether, Sorbitpolyglycidylether, Pentaerythritpolyglycidylether, Propylenglykoldiglycidylether und Polypropylenglykoldiglycidylether, Polyaziridinverbindungen wie 2,2-Bishydroxymethylbutanoltris[3-(1-aziridinyl)propionat), 1,6-Hexamethylendiethylenharnstoff, Diphenylmethan-bis-4,4'-N,N'-diethylenharnstoff, Halogenepoxyverbindungen wie Epichlorhydrin und α-Methylfluorhydrin, Polyisocyanate wie 2,4-Toluylendiisocyanat und Hexamethylendiisocyanat, Alkylencarbonate wie 1,3-Dioxolan-2-on und 4-Methyl-1,3-dioxolan-2-on, polyquaternäre Amine wie Kondensationsprodukte von Dimethylamin mit Epichlorhydrin, Homo- und Copolymere von Diallyldimethylammoniumchlorid sowie Homo- und Copolymerisate von Dimethylaminoethyl(meth)acrylat, die gegebenenfalls mit beispielsweise Methylchlorid quaterniert sind.

Weitere geeignete Vernetzer sind polyvalente Metallionen, die in der Lage sind, ionische Vernetzungen auszubilden. Beispiele für solche Vernetzer sind Magnesium-, Calcium-, Barium- und Aluminiumionen. Diese Vernetzer werden beispielsweise als Hydroxyde, Carbonate oder Hydrogencarbonate der wäßrigen polymerisierbaren Lösung zugesetzt. Ein besonders bevorzugter Vernetzer dieser Art ist Natriumaluminat.

Weitere geeignete Vernetzer sind multifunktionelle Basen, die ebenfalls in der Lage sind, ionische Vernetzungen auszubilden, beispielsweise Polyamine oder deren quaternierte Salze. Beispiele für Polyamine sind Ethylendiamin, Diethylentriamin, Triethylentetramin, Tetraethylenpentamin, Pentaethylenhexamin und Polyethylenimine sowie Polyvinylamine mit Molmassen von jeweils bis zu 4000000.

In einer bevorzugten Ausführungsform der Erfindung werden zwei unterschiedliche Vernetzer eingesetzt, von denen der eine wasserlöslich und der andere wasserunlöslich ist. Der hydrophile Vernetzer, der in der wäßrigen Phase der Reaktionsmischung löslich ist, bewirkt in konventioneller Weise eine relativ gleichmäßige Vernetzung des entstehenden Polymeren, wie es bei der Herstellung eines Superabsorbers üblich ist. Der hydrophobe Vernetzer, der in der polymerisierbaren wäßrigen Mischung unlöslich bzw. darin nur begrenzt löslich ist, reichert sich in der Tensidgrenzschicht zwischen der Gasphase und der polymerisierbaren wäßrigen Phase an. Dadurch wird bei der nachfolgenden Polymerisation die Oberfläche des Schaums stärker vernetzt als der innere Teil des Superabsorberhydrogels. Man erhält dadurch einen Kern-Schale-Aufbau des Schaums unmittelbar bei der Herstellung des Superabsorberschaums. Eine solche starke oberflächliche Vernetzung eines Superabsorberschaums ist bei den bekannten Herstellverfahren des Standes der Technik nur dadurch möglich, daß man einen bereits gebildeten schaumförmigen Superabsorber nachträglich oberflächlich vernetzt. Für diese Nachvernetzung ist bei konventioneller Arbeitsweise ein eigener Prozeßschritt notwendig, der bei dem Verfahren der vorliegenden Erfindung entfallen kann.

Produkte mit einem Kern-Schale-Aufbau zeigen gegenüber homogen vernetzten Proben hinsichtlich der Aufnahmegeschwindigkeit, Verteilungswirkung und Gelstabilität deutlich verbesserte Eigenschaften. Mit Ausnahme polyvalenter Metallionen sind alle oben beschriebenen wasserunlöslichen Vernetzer, die den unterschiedlichen Gruppen zugeordnet werden können, geeignet, um Schäume mit einem Kern-Schale-Aufbau herzustellen, d.h. Schäume, bei denen die gesamte Oberfläche stärker vernetzt ist als die darunter liegende Schicht, die oben als Kernschicht bezeichnet wurde. Besonders bevorzugte hydrophobe Vernetzer sind Diacrylate oder Dimethacrylate oder Divinylether von Alkandiolen mit 2 bis 25 C-Atomen (verzweigt, linear, mit beliebiger Anordnung der OH-Gruppen) wie z.B. 1,3-Propandiol, 1,4-Butandiol, 1,6-Hexandiol, Neopentylglycol, 1,9-Nonandiol oder 1,2-Dodecandiol, Di-, Tri- oder Polypropylenglycoldiacrlyate oder -dimethacrylate, Allylacrylat, Allylmethacrylat, Divinylbenzol, Glycidylacrylat oder Glycidylmethacrylat, Allylglycidylether und Bisglycidylether der oben aufgeführten Alkandiole.

Geeignete hydrophile Vernetzer sind beispielsweise N,N'-Methylenbisacrylamid, Polyethylenglykoldiacrylate oder -dimethacrylate mit einem Molekulargewicht $M_N$ von 200 bis 4000, Divinylharnstoff, Triallylamin, Diacrylate oder Dimethacrylate von Additionsprodukten von 2 bis 400 Mol Ethylenoxid an 1 Mol eines Diols oder Polyols oder das Triacrylat eines Additionsprodukts von 20 Mol Ethylenoxid an 1 Mol Glycerin und Vinylether von Additionsprodukten von 2 bis 400 Mol Ethylenoxid an 1 Mol eines Diols oder Polyols.

Die Monomeren der Gruppe (a) sind in der polymerisierbaren wäßrigen Mischung beispielsweise in Mengen von 10 bis 90 und vorzugsweise 20 bis 85 Gew.-% enthalten. Die Monomeren der Gruppe (b) werden nur gegebenenfalls zur Modifizierung der Superabsorberschäume eingesetzt und können in Mengen bis zu 50, vorzugsweise in Mengen bis zu 20 Gew.-% in der polymerisierbaren wäßrigen Mischung enthalten sein. Die Vernetzer (c) sind in der Reaktionsmischung beispielsweise von 0,001 bis 12 und vorzugsweise von 0,01 und 8 Gew.-% vorhanden.

Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wäßrige Mischung ist möglich. Die Polymerisation kann allerdings auch in Abwesenheit von Initiatoren der obengenannten Art durch Einwirkung energiereicher Strahlung in Gegenwart von Photoinitiatoren ausgelöst werden.

Als Polymerisationsinitiatoren können sämtliche unter den Polymerisationsbedingungen in Radikale zerfallende Verbindungen eingesetzt werden, z.B. Peroxide, Hydroperoxide, Wasserstoffperoxid, Persulfate, Azoverbindungen und die sogenannten Redoxkatalysatoren. Bevorzugt ist der Einsatz von wasserlöslichen Initiatoren. In manchen Fällen ist es vorteilhaft, Mischungen verschiedener Polymerisationsinitiatoren zu verwenden, z.B. Mischungen aus Wasserstoffperoxid und Natrium- oder Kaliumperoxodisulfat. Mischungen aus Wasserstoffperoxid und Natriumperoxodisulfat können in jedem beliebigen Verhältnis verwendet werden. Geeignete organische Peroxide sind beispielsweise Acetylacetonperoxid, Methylethylketonperoxid, tert.-Butylhydroperoxid, Cumolhydroperoxid, tert.-Amylperpivalat, tert.-Butylperpivalat, tert.-Butylperneohexanoat, tert.-Butylperisobutyrat, tert.-Butylper-2-ethylhexanoat, tert.-Butylperisononanoat, tert.-Butylpermaleat, tert.-Butylperbenzoat, Di-(2-ethylhexyl)-peroxidicarbonat, Dicyclohexylperoxydicarbonat, Di-(4-tert.-butylcyclohexyl)peroxidicarbonat, Dimyristil-peroxidicarbonat, Diacetylperoxydicarbonat, Allylperester, Cumylperoxyneodecanoat, tert.-Butylper-3,5,5-tri-methylhexanoat, Acetylcyclohexylsulfonylperoxid, Dilaurylperoxid, Dibenzoylperoxid und tert.-Amylperneodekanoat. Besonders geeignete Polymerisationsinitiatoren sind wasserlösliche Azostarter, z.B. 2,2'-Azobis-(2-amidinopropan)dihydrochlorid, 2,2'-Azobis-(N,N'-dimethylen)isobutyramidin-dihydrochlorid, 2-(Carbamoylazo)isobutyronitril, 2,2'-Azobis[2-(2'-imidazolin-2-yl)propan]dihydrochlorid und 4,4'-Azobis-(4-cyanovaleriansäure). Die genannten Polymerisationsinitiatoren werden in üblichen Mengen eingesetzt, z.B. in Mengen von 0,01 bis 5, vorzugsweise 0,1 bis 2,0 Gew.-%, bezogen auf die zu polymerisierenden Monomeren.

Als Initiatoren kommen außerdem Redoxkatalysatoren in Betracht. Die Redoxkatalysatoren enthalten als oxidierende Komponente mindestens eine der oben angegebenen Perverbindungen und als reduzierende Komponente beispielsweise Ascorbinsäure, Glukose, Sorbose, Ammonium- oder Alkalimetall-hydrogensulfit, -sulfit, -thiosulfat, -hyposulfit, -pyrosulfit oder -sulfid, Metallsalze, wie Eisen-II-ionen oder Silberionen oder Natriumhydroxymethylsulfoxylat. Vorzugsweise verwendet man als reduzierende Komponente des Redoxkatalysators Ascorbinsäure oder Natriumsulfit. Bezogen auf die bei der Polymerisation eingesetzte Menge an Monomeren verwendet man beispielsweise $3.10^{-6}$ bis 1 Mol-% der reduzierenden Komponente des Redoxkatalysatorsystems und 0,001 bis 5,0 Mol-% der oxidierenden Komponente des Redoxkatalysators.

Wenn man die Polymerisation durch Einwirkung energiereicher Strahlung auslöst, verwendet man üblicherweise als Initiator sogenannte Photoinitiatoren. Hierbei kann es sich beispielsweise um sogenannte α-Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanthon-Derivate, Cumarin-Derivate, Benzoinether und deren Derivate, Azoverbindungen wie die oben genannten Radikalbildner, substituierte Hexaarylbisimidazole oder Acylphosphinoxide. Beispiele für Azide sind: 2-(N,N-Dimethylamino)-ethyl-4-azidocinnamat, 2-(N,N-Dimethylamino)-ethyl-4-azidonaphthylketon, 2-(N,N-Dimethylamino)-ethyl-4-azidobenzoat, 5-Azido-1-naphthyl-2'-(N,N-dimethylamino)ethylsulfon, N-(4-Sulfonylazidophenyl)maleinimid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclohexanon und 2,6-Bis-(p-azidobenzyliden)-4-methylcyclohexanon. Die Photoinitiatoren werden, falls sie eingesetzt werden, üblicherweise in Mengen von 0,01 bis 5 Gew.-%, bezogen auf die zu polymerisierenden Monomeren angewendet.

Die polymerisierbaren wäßrigen Mischungen enthalten als Komponente (e) 0,1 bis 20 Gew.-% mindestens eines Tensids. Die Tenside sind für die Herstellung und die Stabilisierung des Schaums von entscheidender Bedeutung. Man kann anionische, kationische oder nichtionische Tenside oder Tensidmischungen verwenden, die miteinander verträglich sind. Man kann niedermolekulare oder auch polymere Tenside einsetzen, wobei sich Kombinationen unterschiedlicher oder auch gleichartiger Typen von Tensiden als vorteilhaft herausgestellt haben. Nichtionische Tenside sind beispielsweise Additionsprodukte von Alkylenoxiden, insbesondere Ethylenoxid, Propylenoxid und/oder Butylenoxid an Alkohole, Amine, Phenole, Naphthole oder Carbonsäuren. Vorteilhaft setzt man als Tenside Additionsprodukte von Ethylenoxid und/oder Propylenoxid an mindestens 10 C-Atome enthaltende Alkohole ein, wobei die Additionsprodukte pro Mol Alkohol 3 bis 200 Mol Ethylenoxid und/oder Propylenoxid angelagert enthalten. Die Additionsprodukte enthalten die Alkylenoxid-Einheiten in Form von Blöcken oder in statistischer Verteilung. Beispiele für nichtionische Tenside sind die Additionsprodukte von 7 Mol Ethylenoxid an 1 Mol Talgfettalkohol, Umsetzungsprodukte von 9 Mol Ethylenoxid mit 1 Mol Talgfettalkohol und Additionsprodukte von 80 Mol Ethylenoxid an 1 Mol Talgfettalkohol. Weitere handelsübliche nichtionische Tenside bestehen aus Umsetzungsprodukten von Oxoalkoholen oder Ziegler-Alkoholen mit 5 bis 12 Mol Ethylenoxid pro Mol Alkohol, insbesondere mit 7 Mol Ethylenoxid. Weitere handelsübliche nichtionische Tenside werden durch Ethoxylierung von Rizinusöl erhalten. Pro Mol Rizinusöl werden beispielsweise 12 bis 80 Mol Ethylenoxid angelagert. Weitere handelsübliche Produkte sind beispielsweise die Umsetzungsprodukte von 18 Mol Ethylenoxid mit 1 Mol Talgfettalkohol, die Additionsprodukte von 10 Mol Ethylenoxid an 1 Mol eines $C_{13}/C_{15}$-Oxoalkohols, oder die Umsetzungsprodukte von 7 bis 8 Mol Ethylenoxid an 1 Mol eines $C_{13}/C_{15}$-Oxoalkohols. Weitere geeignete nichtionische Tenside sind Phenolalkoxylate wie beispielsweise p-tert.-Butylphenol, das mit 9 Mol Ethylenoxid umgesetzt ist, oder Methylether von Umsetzungsprodukten aus 1 Mol eines $C_{12}$- bis $C_{18}$-Alkohols und 7,5 Mol Ethylenoxid.

Die oben beschriebenen nichtionischen Tenside können beispielsweise durch Veresterung mit Schwefelsäure in die entsprechenden Schwefelsäurehalbester überführt werden. Die Schwefelsäurehalbester werden in Form der Alkalimetall- oder Ammoniumsalze als anionische Tenside eingesetzt. Als anionische Tenside eignen sich beispielsweise Alkalimetall- oder Ammoniumsalze von Schwefelsäurehalbestern von Additionsprodukten von Ethylenoxid und/oder Propylenoxid an Fettalkohole, Alkalimetall- oder Ammoniumsalze von Alkylbenzolsulfonsäure oder von Alkylphenolethersulfaten. Produkte der genannten Art sind im Handel erhältlich. Beispielsweise sind das Natriumsalz eines Schwefelsäurehalbesters eines mit 106 Mol Ethylenoxid umgesetzten $C_{13}/C_{15}$-Oxoalkohols, das Triethanolaminsalz von Dodecylbenzolsulfonsäure, das Natriumsalz von Alkylphenolethersulfaten und das Natriumsalz des Schwefelsäurehalbesters eines Umsetzungsprodukts von 106 Mol Ethylenoxid mit 1 Mol Talgfettalkohol handelsübliche anionische Tenside. Weitere geeignete anionische Tenside sind Schwefelsäurehalbester von $C_{13}/C_{15}$-Oxoalkoholen, Paraffinsulfonsäuren wie $C_{15}$-Alkylsulfonat, alkylsubstituierte Benzolsulfonsäuren und alkylsubstituierte Naphthalinsulfonsäuren wie Dodecylbenzolsulfonsäure und Di-n-butylnaphthalinsulfonsäure sowie Fettalkoholphosphate wie $C_{15}/C_{18}$-Fettalkoholphosphat. Die polymerisierbare wäßrige Mischung kann Kombinationen aus einem nichtionischen Tensid und einem anionischen Tensid oder Kombinationen aus nichtionischen Tensiden oder Kombinationen aus anionischen Tensiden enthalten. Auch kationische Tenside sind geeignet. Beispiele hierfür sind die mit Dimethylsulfat quaternierten Umsetzungsprodukte von 6,5 Mol Ethylenoxid mit 1 Mol Oleylamin, Distearyldimethylammoniumchlorid, Lauryltrimethylammoniumchlorid, Cetylpyridiniumbromid und mit Dimethylsulfat quaternierter Stearinsäuretriethanolaminester, der bevorzugt als kationisches Tensid eingesetzt wird.

Der Tensidgehalt der polymerisierbaren wäßrigen Mischung beträgt vorzugsweise 0,5 bis 10 Gew.-%. In den meisten Fällen weisen die polymerisierbaren wäßrigen Mischungen einen Tensidgehalt von 1,5 bis 8 Gew.-% auf.

Die polymerisierbaren wäßrigen Mischungen können als Komponente (f) gegebenenfalls mindestens einen Lösevermittler enthalten. Hierunter sollen mit Wasser mischbare organische Lösemittel verstanden werden, z.B. Alkohole, Glykole, Polyethylenglykole bzw. davon abgeleitete Monoether, wobei die Monoether keine Doppelbindungen im Molekül enthalten. Geeignete Ether sind Methylglykol, Butylglykol, Butyldiglykol, Methyldiglykol, Butyltriglykol, 3-Ethoxy-1-propanol und Glycerinmonomethylether.

Die polymerisierbaren wäßrigen Mischungen enthalten 0 bis 50 Gew.-% mindestens eines Lösevermittlers. Falls Lösevermittler eingesetzt werden, beträgt ihr Gehalt in der polymerisierbaren wäßrigen Mischung vorzugsweise 1 bis 25 Gew.-%.

Die polymerisierbare wäßrige Mischung kann gegebenenfalls Verdicker, Schaumstabilisatoren, Polymerisationsregler, Füllstoffe und Zellkeimbildner enthalten. Verdicker werden beispielsweise zur Optimierung der Schaumstruktur und zur Verbesserung der Schaumstabilität eingesetzt. Man erreicht damit, daß der Schaum während der Polymerisation nur geringfügig schrumpft. Als Verdickungsmittel kommen alle hierfür bekannten natürlichen und synthetischen Polymeren in Betracht, die die Viskosität eines wäßrigen Systems stark erhöhen. Hierbei kann es sich um wasserquellbare oder wasserlösliche synthetische und natürliche Polymere handeln.

Als Verdicker sind auch pulverförmige Superabsorber geeignet. Eine ausführliche Übersicht über Verdicker findet man beispielsweise in den Veröffentlichungen von R.Y. Lochhead und W.R. Fron, Cosmetics & Toiletries, 108, 95-135 (Mai 1993) und M.T. Clarke, "Rheological Additives" in D. Laba (ed.) "Rheological Properties of Cosmetics and Toiletries", Cosmetic Science and Technology Series, Vol. 13, Marcel Dekker Inc., New York 1993.

Als Verdicker in Betracht kommende wasserquellbare oder wasserlösliche synthetische Polymere sind beispielsweise hochmolekulare Polymerisate der oben unter (a) beschriebenen Säuregruppen enthaltenden monoethylenisch ungesättigten Monomeren. Solche Verdicker sind beispielsweise hochmolekulare Homopolymerisate von Acrylsäure und/oder Methacrylsäure oder geringfügig vernetzte Copolymerisate aus Acrylsäure und/oder Methacrylsäure und einer Verbindung, die mindestens 2 ethylenisch ungesättigte Doppelbindungen enthält, z.B. Butandioldiacrylat. Außerdem eignen sich hochmolekulare Polymerisate von Acrylamid und Methacrylamid oder Copolymerisate aus Acrylsäure und Acrylamid mit Molmassen von mehr als 1 Million. Solche Copolymerisate sind als Verdickungsmittel bekannt. Auch hochmolekulare Polyethylenglykole oder Copolymerisate aus Ethylenglykol und Propylenglykol sowie hochmolekulare Polysaccharide wie Stärke, Guarkernmehl, Johannisbrotkernmehl oder Derivate von Naturstoffen wie Carboxymethylcellulose Hydroxyethylcellulose, Hydroxymethylcellulose, Hydroxypropylcellulose und Cellulose Mischether sind bekannte Verdicker. Eine weitere Gruppe von Verdickern sind wasserunlösliche Produkte, wie feinteiliges Siliciumdioxid, pyrogene Kieselsäuren, Fällungskieselsäuren in hydrophilen oder hydrophoben Modifikationen, Zeolithe, Titandioxid, Cellulosepulver, oder andere von Superabsorbern verschiedene feinteilige Pulver von vernetzten Polymerisaten. Die polymerisierbaren wäßrigen Mischungen können die Verdicker in Mengen bis zu 30 Gew.-% enthalten. Falls solche Verdickungsmittel überhaupt eingesetzt werden, sind sie in Mengen von 0,1, vorzugsweise 0,5 bis 20 Gew.-% in der polymerisierbaren wäßrigen Mischung enthalten.

Um die Schaumstruktur zu optimieren, kann man gegebenenfalls Kohlenwasserstoffe mit mindestens 5 C-Atomen im Molekül zu der wäßrigen Reaktionsmischung zusetzen. Geeignete Kohlenwasserstoffe sind beispielsweise Pentan, Cyclopentan, Hexan, Cyclohexan, Heptan, Octan, Isooctan, Decan und Dodecan. Die in Betracht kommenden aliphatischen Kohlenwasserstoffe können geradkettig, verzweigt oder zyklisch sein und haben eine Siedetemperatur, die oberhalb der Temperatur der wäßrigen Mischung während des Schäumens liegt. Die aliphatischen Kohlenwasserstoffe erhöhen die Standzeit der noch nicht polymerisierten geschäumten wäßrigen Reaktionsmischung. Dadurch wird das Handling der noch nicht polymerisierten Schäume erleichtert und die Prozeßsicherheit erhöht. Die Kohlenwasserstoffe werden in Mengen von 0 bis 10 Gew.-%, bezogen auf die polymerisierbare wäßrige Mischung eingesetzt. Im Fall ihres Einsatzes betragen die bevorzugt in der wäßrigen Mischung vorliegenden Mengen 0,1 bis 5 Gew.-%.

Um die Eigenschaften der Superabsorber zu variieren, beispielsweise die Aufnahmegeschwindigkeit und die Aufnahmekapazität von Wasser, kann es von Vorteil sein, der wäßrigen Reaktionsmischung einen Polymerisationsregler oder eine Mischung mehrerer Polymerisationsregler zuzusetzen. Geeignete Polymerisationsregler sind beispielsweise Ameisensäure, Thioverbindungen wie 2-Mercaptoethanol, Mercaptopropanol, Mercaptobutanol, Dodecylmercaptan, Thioglykolsäure oder Amine wie Triethylamin, Morpholin oder Piperidin. Die Mengen an Polymerisationsregler können bis zu 10 Gew.-%, bezogen auf die eingesetzten Monomeren, betragen. Falls Polymerisationsregler eingesetzt werden, verwendet man vorzugsweise 0,1 bis 5 Gew.-%, bezogen auf die Monomeren.

Die unter (g) angegebenen fakultativ zu verwendenden Bestandteile können einzeln oder in Mischung bei der Herstellung der erfindungsgemäßen Polymerisate eingesetzt werden. Man kann jedoch auch in Abwesenheit von Verdickern, Schaumstabilisatoren, Füllstoffe, Zellkeimbildner und Polymerisationsreglern arbeiten.

Bei der erfindungsgemäßen Herstellung von wasserabsorbierenden, schaumförmigen, vernetzten Polymerisaten wird in einer ersten Verfahrensstufe die oben beschriebene polymerisierbare wäßrige Mischung geschäumt. Zu diesem Zweck wird die wässrige Monomerphase unter einem Druck von 2 bis 400 bar, vorzugsweise 5 bis 40 bar mit einem gegenüber Radikalen inerten Gas beaufschlagt, wobei sich beispielsweise mindestens 1,0 g des inerten Gases pro kg der polymerisierbaren Mischung lösen. Besonders bevorzugt ist eine Arbeitsweise, bei der man unter den genannten Druckbedingungen eine mit inerten Gasen gesättigte, polymerisierbare Mischung herstellt. Für das erfindungsgemäße Verfahren geeignete inerte Gase sind beispielsweise Kohlendioxid, Stickstoff und Distickstoffmonoxid. Man kann z.B. auch Mischungen aus Kohlendioxid und Stickstoff in jedem beliebigen Verhältnis verwenden. Bevorzugt eingesetztes Gas ist Kohlendioxid. Bei Verwendung von Kohlendioxid als gegenüber Radikalen inertem Gas benötigt man beispielsweise zum Aufschäumen von 1 kg der wäßrigen polymerisierbaren Monomermischung mindestens 2,0 g Kohlendioxid bevorzugt 8 - 30 g. Das Lösen der inerten Gase in den wäßrigen, polymerisierbaren Mischungen, vorzugsweise die Herstellung gesättigter wäßriger, polymerisierbarer Mischungen kann im Batch-Betrieb oder auch kontinuierlich erfolgen. Anschließend wird die gasgesättigte Monomerphase auf Normaldruck entspannt. Dabei bildet sich ein feinteiliger, geschlossenzelliger Monomerschaum, der längere Zeit stabil ist. Typische Standzeiten variieren zwischen 10 min und mehreren Stunden.

Die Entspannung kann in einem oder auch in mehreren Drucksprüngen erfolgen. Im einfachsten Falle strömt die unter Druck stehende Reaktionsmischung durch eine Düse aus. Zur Optimierung der Monomerschaumstruktur, kann es vorteilhaft sein, spezielle Techniken anzuwenden. So kann eine Mischdüse Verwendung finden, die in den austretenden Strom der Reaktionsmischung einen zusätzlichen Gasstrom einmischt, wobei das hier zugemischte Gas identisch oder unterschiedlich sein kann zu dem zur Sättigung verwendeten. Durch das Zumischen des Gases während der Entspannung werden Keime für die Freisetzung des gelösten Gases geschaffen, wodurch die Struktur des Monomerschaums gezielt beeinflußt werden kann. Eine andere Möglichkeit, Keime bei der Entspannung zu erzeugen besteht darin, die Reaktionsmischung unmittelbar vor der Entspannung durch eine Einheit laufen zulassen, die einen kavitierenden Rührer enthält. Fernerhin kann es vorteilhaft sein, den austretenden Flüssigkeitsstrom gegen einen Prallkörper zu lenken, wobei die Schaumbildung beim Auftreffen auf den Prellkörper erfolgt. Diese Vorgehensweise hat den Vorteil, daß der Schaum eine homogenere Porengrößenverteilung erhält. Wird statt dessen der Flüssigkeitsstrahl direkt in den Schaum eingeführt, so werden durch den Sog des Flüssigkeitsstrahls zusätzliche Gasblasen in den Schaum eingetragen, wodurch sich die Blasengrößenverteilung in der Regel verbreitert.

Zweckmäßigerweise geht man so vor, daß zunächst alle wasserlöslichen Komponenten in Wasser gelöst werden und daß man erst danach die wasserunlöslichen Stoffe zusetzt. Je nach eingesetztem Initiator kann es auch von Vorteil sein, den Initiator erst am Ende des Sättigungsprozesses zuzusetzen. Die Konsistenz der Schäume kann in einem weiteren Bereich variiert werden. So ist es möglich, leichte fließfähige Schäume oder aber steife, schnittfeste noch nicht polymerisierte Schäume herzustellen. Ebenso kann man die mittlere Größe der Gasblasen, ihre Größenverteilung und ihre Anordnung in der Flüssigkeitsmatrix durch die Auswahl der Tenside, der Lösevermittler, Verdicker und Schaumstabilisatoren, Zellkeimbildner, der Temperatur, des Sättigungsdrucks und der oben geschilderten Keimbildungsmaßnahmen in einem weiten Bereich variieren, so daß man in einfacher Weise Dichte oder Wandstärke des Matrixmaterials einstellen kann. Die Temperaturen der polymerisierbaren wäßrigen Mischung liegen während des Schäumvorgangs in dem Bereich von -20 bis 100, vorzugsweise -5 bis +40°C.

Durch geeignete Wahl des eingesetzten Gases und des Sättigungsdrucks kann man in einfacher Weise gezielt bestimmte Schaumdichten einstellen. Da die erhaltenen Monomerschäume über längere Zeiträume, z.B. bis zu 6 Stunden stabil und einfach zu handhaben sind, können sie für die nachfolgende Polymerisation in eine geeignete Form gebracht werden, um die für eine bestimmte Anwendung gewünschten Formkörper herzustellen. Der bei der Formgebung der geschäumten polymerisierbaren wäßrigen Mischung möglicherweise anfallende Abfallschaum kann ohne weiteres in den Prozeß zurückgeführt werden. Das geschäumte polymerisierbare Material kann beispielsweise in der gewünschten Stärke auf ein temporäres Trägermaterial, das vorteilhafterweise mit einer Antihaftbeschichtung ausgestattet ist, aufgetragen werden. Man kann beispielsweise den Schaum auf eine Unterlage aufrakeln. Eine andere Möglichkeit besteht darin, die polymerisierbare schaumförmige wäßrige Mischung in Formen einzufüllen, die ebenfalls antihaftbeschichtet sind und den Schaum darin auszupolymerisieren.

Da die geschäumte polymerisierbare wäßrige Mischung eine lange Standzeit aufweist, eignet sich diese Mischung auch für die Herstellung von Verbundmaterialien. So kann beispielsweise der nach der Schaumerzeugung hergestellte polymerisierbare Schaum auf ein permanentes Trägermaterial aufgebracht werden, z.B. Folien aus Polymeren (z.B. Folien aus Polyethylen, Polypropylen oder Polyamid) oder Metallen, Vliesen, Fluff, Tissues, Gewebe, natürliche oder synthetische Fasern, oder auf andere Schäume. Bei der Herstellung von Verbundmaterialien kann es unter Umständen auch vorteilhaft sein, den polymerisierbaren Schaum in Gestalt von bestimmten Strukturen oder in unterschiedlicher Schichtdicke auf ein Trägermaterial aufzubringen. Es ist jedoch auch möglich, den polymerisierbaren Schaum auf Fluff-Schichten aufzutragen und sie so zu imprägnieren, daß der Fluff nach der Polymerisation integraler Bestandteil des Schaums ist. Die in der ersten Verfahrensstufe erhältliche geschäumte polymerisierbare wäßrige Mischung kann auch zu großen Blöcken geformt und polymerisiert werden. Die Blöcke können nach der Polymerisation zu kleineren Formkörpern geschnitten oder gesägt werden. Man kann auch sandwichartige Strukturen herstellen, indem man eine geschäumte polymerisierbare wäßrige Mischung auf eine Unterlage aufträgt, die schaumförmige Schicht mit einer Folie, Vliesen, Tissues, Geweben, Fasern oder anderen Schäumen gegebenenfalls aus einem anderen Material als die zunächst verwendete Unterlage abdeckt und wiederum Schaum aufträgt und gegebenenfalls mit einer weiteren Folie, Vliesen, Tissues, Geweben, Fasern oder anderen Schäumen abdeckt. Der Verbund wird dann in der zweiten Verfahrensstufe der Polymerisation unterworfen. Man kann jedoch auch sandwichartige Strukturen mit weiteren Schaumschichten herstellen.

In der zweiten Stufe des Verfahrens zur Herstellung der erfindungsgemäßen superabsorbierenden Schäume erfolgt die Polymerisation der geschäumten polymerisierbaren wäßrigen Mischung. Die Polymerisation kann je nach verwendetem Initiator durch Temperaturerhöhung, durch Lichteinwirkung, durch Bestrahlen mit Elektronenstrahlen oder auch durch Temperaturerhöhung und Lichteinwirkung erfolgen. Um die Temperatur der geschäumten polymerisierbaren wäßrigen Mischung zu erhöhen, kann man alle in der Technik üblichen Verfahren anwenden, beispielsweise den Schaum mit heizbaren Platten in Kontakt bringen, Einwirkung von Infrarotbestrahlung auf den polymerisierbaren Schaum oder Beheizen mit Hilfe von Mikrowellen. Erfindungsgemäße Schaumschichten mit einer Schichtdicke von bis zu etwa 1 Millimeter stellt man beispielsweise durch einseitiges Erwärmen oder insbesondere durch einseitiges Bestrahlen her. Falls dickere Schichten eines Schaums hergestellt werden sollen, z.B. Schäume mit Dicken von mehreren Zentimetern, ist die Erwärmung des polymerisierbaren geschäumten Materials mit Hilfe von Mikrowellen besonders vorteilhaft, weil auf diesem Wege eine relativ gleichmäßige Erwärmung erreicht werden kann. Die Polymerisation erfolgt dabei beispielsweise bei Temperaturen von 20 bis 180, vorzugsweise in dem Bereich von 20 bis 100°C.

Schaumschichten von mittleren Schichtdicken, d.h. mit einer Dicke im Bereich von etwa 1 Millimeter bis etwa 2 Zentimeter, wie z.B. von etwa 2 Millimeter bis etwa 1 Zentimeter, stellt man vorzugsweise in folgender Weise her: Anstatt die Polymerisation nur einflächig zu initiieren, bewirkt man eine beidflächige Initiierung, indem man eine Schicht der erfindungsgemäß geschäumten Masse einer beidflächigen Wärmebehandlung und/oder Bestrahlung mit Licht aussetzt. Die Behandlung beider Flächen der Schaumschicht kann erfindungsgemäß synchron oder in beliebiger zeitlicher Reihenfolge asynchron oder zeitlich versetzt erfolgen. Man kann beispielsweise die Wärmebehandlung beider Teilflächen einer Schaumschicht gleichzeitig oder zeitlich versetzt einmalig oder mehrmals pro Teilfläche durchführen. Ebenso kann man bei der Bestrahlung mit Licht verfahren. Es besteht aber auch die Möglichkeit, jede Teilfläche sowohl mit Wärme als auch mit Licht zu behandeln, wobei Wärme und Licht gleichzeitig oder in beliebiger Abfolge, einmalig oder mehrfach auf die gleiche Teilfläche der Schaumschicht einwirken können. Am zweckmäßigsten ist jedoch gewöhnlich die einmalige Anwendung von Wärme und/oder Licht je Teilfläche der Schaumschicht.

Da die Wärmebehandlung zweckmäßigerweise durch Kontaktheizung erfolgt und das dafür verwendete Trägermaterial gewöhnlich lichtundurchlässig ist, wird die beidseitige Polymerisationsinitiation am zweckmäßigsten durch Kontaktheizen einer Teilfläche und, beispielsweise simultanes, Bestrahlen der gegenüberliegenden Teilfläche durchgeführt. Diese Verfahrensvariante sowie das beidseitige Kontaktheizen eignen sich insbesondere zur Herstellung von Verbundmaterialien.

Die Wärmebehandlung erfolgt bei der beidflächigen Polymerisationsinitiation gewöhnlich in einem Bereich von etwa 50 bis etwa 200°C, vorzugsweise bei etwa 80 bis etwa 160°C. Typische Kontaktzeiten liegen dabei bei etwa 0,5 bis etwa 25 Minuten je Teilfläche der Schaumschicht, vorzugsweise bei etwa 2 bis etwa 15 Minuten. Zur Bestrahlung verwendet man vorzugsweise Licht aus dem UV/VIS-Bereich, d.h. Licht aus dem ultravioletten oder sichtbaren Bereich des Spektrums, wie z.B. Licht mit einer Wellenlänge im Bereich von etwa größer 200 nm bis etwa 750 nm, beispielsweise etwa 250 nm bis etwa 700 nm, wie etwa UV-A-Strahlung der Wellenlänge 315 bis 400 nm. Die Dauer der Bestrahlung kann ebenfalls im Bereich von etwa 0,1 bis etwa 25 Minuten, vorzugsweise bei etwa 0,5 bis 10 Minuten, je Teilfläche der Schaumschicht liegen.

Bei kombinierter Wärmebehandlung und Bestrahlung derselben oder gegenüberliegender Teilflächen der Schaumschicht kann die jeweilige Dauer von Wärmebehandlung und Bestrahlung gleich oder verschieden sein. In Abhängigkeit von Zusammensetzung und Dicke der Schaumschicht, Art und Menge der verwendeten Polymerisationsinitiatoren, Intensität und Wellenlänge des Lichts sowie Temperatur der Kontaktheizvorrichtung und anderer Kriterien kann es aber von Vorteil sein, Wärmebehandlung und Bestrahlung über unterschiedlich lange Zeitintervalle durchzuführen. Die gewählten Zeitintervalle können z.B. zeitlich aufeinander folgen. Beispielsweise kann auf eine z.B. 3-minütige Erwärmung der ersten Teilfläche eine z.B. 2-minütige Bestrahlung der gegenüberliegenden zweiten Teilfläche folgen. Daran kann sich gegebenenfalls eine z.B. 2-minütige Wärmebehandlung der ersten und/oder der zweiten Teilfläche anschließen. Diesen Behandlungsrhythmus kann man gegebenenfalls unter Beibehaltung oder Veränderung der gewählten Zeitintervalle einmal oder mehrfach wiederholen. Die gewählten Zeitintervalle können sich aber auch überlappen. Beispielsweise kann man dabei die Bestrahlung nur über einen Teil des Wärmebehandlungsintervalls aufrechterhalten. So kann man beispielsweise die erste Teilfläche der Schaumschicht z.B. 2 Minuten erhitzen und anschließend z.B. weitere 4 Minuten erhitzen und synchron dazu die gegenüberliegende Fläche 4 Minuten bestrahlen. Ebenso ist es vorstellbar, die beiden Teilflächen zunächst z.B. 3 Minuten synchron zu erhitzen bzw. zu bestrahlen und anschließend die Wärmebehandlung der einen Teilfläche z.B. 2 Minuten fortzusetzen, nachdem man die Bestrahlung der anderen Teilfläche beendet hat. Auch diese Behandlungsrhythmen kann man gegebenenfalls unter Beibehaltung oder Veränderung der gewählten Zeitintervalle einmal oder mehrfach wiederholen.

Bei Initiierung der Polymerisation durch Lichteinwirkung auf das geschäumte polymerisierbare Material kann man alle konventionellen Belichtersysteme anwenden, sofern ihr Emissionsspektrum an den eingesetzten Photoinitiator adaptiert ist. Bei einem Start der Polymerisation durch Belichten wird vorteilhaft eine Kombination eines Photoinitiators eines thermischen Initiators oder/und aber ein Photoinitator eingesetzt, der auch als thermischer Initiator wirken kann, z.B. Azoinitiatoren. Da sich der Schaum während der Polymerisation durch die hohe Polymerisationswärme stark erwärmt, wird auf diese Weise ein besonders schneller und effektiver Ablauf der Polymerisationsreaktion erreicht. Bei der Initiierung durch Lichteinwirkung liegt die Polymerisationstemperatur in dem Bereich von 0 bis 150, vorzugsweise 10 bis 100°C.

Während der Polymerisation ändert sich die Dichte des Schaums nur unwesentlich. Jedoch öffnet sich die primär geschlossenzellige Struktur dergestalt, daß die Membranen zwischen benachbarten Schaumblasen aufreißen und sich somit Fenster (Durchgänge) zwischen den einzelnen Poren ausbilden. Durch diesen Vorgang wird als Endprodukt der Polymerisation ein weitgehend, bevorzugt jedoch vollständig offenzelliger Schaum erhalten. Die Polymersationsreaktion sowie dieser Öffnungsmechanismus wird durch die Starttemperatur, die Initiierungstechnik oder die Wärmeabfuhr beeinflußt. Die Polymerisationstemperatur wird vorzugsweise dahingehend kontrolliert, daß ein Sieden der polymerisierbaren wäßrigen Mischung vermieden wird. Mit fortschreitender Polymerisation tritt eine Verfestigung des Schaums infolge zunehmender Gelbildung ein.

Nach der Polymerisation haben die vorliegenden Schäume einen Wassergehalt zwischen 5% und 80 %. Im Prinzip können durch den Einsatz von Alkanolaminen Schäume erhalten werden, die auch im getrockneten Zustand flexibel sind. Da die Schäume jedoch hygroskopisch sind und aus der Luft ohnehin Feuchtigkeit aufnehmen, ist es sinnvoll, eine Restfeuchte im Bereich von 0,1 - 20, bevorzugt 1 - 15 Gew.-% im Schaum zu belassen. Ferner kann es je nach Zusammensetzung des Schaums und dem beabsichtigten Einsatzgebiet auch sinnvoll sein, einen davon abweichenden Feuchtegehalt im Schaum einzustellen.

Der Schaum kann mit Hilfe konventioneller Techniken getrocknet werden, beispielsweise durch Erhitzen mit einem heißen Gasstrom, durch Anlegen von Vakuum, durch Infrarotbestrahlung oder durch Erhitzen mit Mikrowellenstrahlung. Die Mikrowellenstrahlung erweist sich auch hier wiederum beim Trocknen von großvolumigen Formkörpern als vorteilhaft. Bei der Trocknung sollte die Temperatur kleiner 180°C, bevorzugt kleiner 120°C liegen. Es kann vorteilhaft sein mit einem Gasstrom zu trocknen, der einen definierten Feuchtegehalt aufweist (bis hin zum Einsatz von Wasserdampf), so daß auf diesem Weg der Schaum nur bis zu einem definierten Feuchtegehalt getrocknet wird.

Gegenüber den bisher bekannten schaumförmigen Superabsorbern ist ein wesentlicher Vorteil der erfindungsgemäßen Schäume darin zu sehen, daß sie eine verbesserte Fähigkeit aufweisen, wässrige Flüssigkeiten über den gesamten Absorptionskern eines Hygieneartikels zu verteilen. Verfahrenstechnisch bietet die erfindungsgemäße Herstellung der Schäume den Vorteil, daß die Temperaturkontrolle als kritischer Verfahrensparameter bei der Schaumherstellung wesentlich vereinfacht wird und somit die Prozeßsicherheit und die Reproduzierbarkeit erhöht wird.

Nach dem erfindungsgemäßen Verfahren erhält man einen überwiegend offenzelligen Superabsorberschaum, der vorzugsweise zu mindestens 80 % offenzellig ist. Die so herstellbaren wasserabsorbierenden, schaumförmigen, vernetzten Polymerisate haben eine freie Aufnahmegeschwindigkeit (FAG) für eine 0,9 gew.-%ige wäßrige Kochsalzlösung von 4,0 bis 100 g/g sec..Sie verfügen über eine Vertikal Wicking Time (VWT = Zeit für die Ausbreitung einer 0,9 gew.-%igen wäßrigen Kochsalzlösung in einem Schaumstoff in vertikaler Richtung) für eine Höhe von 4 cm von 0,2 bis 120 Sekunden.

Wie oben bereits angegeben wurde, kann eine inhomogene Vernetzungsdichte bei den erfindungsgemäßen Superabsorberschäumen bereits während der Herstellung erzeugt werden. Dies ist besonders vorteilhaft, wenn man als Monomere der oben beschriebenen Komponenten

(a) Acrylsäure, Methacrylsäure, Vinylsulfonsäure, Acrylamidopropansulfonsäure oder deren Mischungen und

(c) eine Mischung aus mindestens einem wasserlöslichen und mindestens einem wasserunlöslichen Vernetzer einsetzt.

Dennoch kann es wünschenswert sein, den Vernetzungsgrad des Schaumes nachträglich zu verändern. Um dieses Ziel zu erreichen, kann man beispielsweise während der Polymerisation durch den Zusatz geeigneter Monomere in das Gel latente Vernetzungsstellen einbauen, die unter den Bedingungen der Schaumherstellung nicht zu Vernetzungsreaktionen führen, jedoch unter speziellen Bedingungen, die nachträglich angewandt werden können, z.B. durch stark erhöhte Temperatur, in der Lage sind, weitere Vernetzungspunkte in der Gelstruktur zu bilden. Als Beispiele für solche Monomere kann der Einbau von Hydroxylgruppen enthaltenden Verbindungen dienen, die bei höherer Temperatur, d.h. bei Temperaturen oberhalb von 150°C in der Lage sind, mit den Carboxylgruppen in der Schaumstruktur zu reagieren. Geeignete Verbindungen, die latente Vernetzungsstellen aufweisen, sind beispielsweise Hydroxyethylacrylat, Hydroxypropylacrylat, Hydroxybutylacrylat, Monoacrylsäureester des Glycerins, Monoacrylate oder Monomethacrylate von Polyethylenglykolen mit mindestens 2 Ethylenglykoleinheiten, Monoacrylate oder Monomethacrylate von Polypropylenglykolen mit mindestens 2 Propylenglykoleinheiten und Monomethacrylate von mehrwertigen Alkoholen, z.B. Hydroxybutylmethacrylat, Hydroxypropylmethacrylat, Hydroxyethylmethacrylat oder Glycerinmonomethacrylat.

Als weitere Möglichkeit einer homogenen Nachvernetzung bietet sich der nachträgliche Zusatz von Vernetzungsreagenzien an, d.h. Verbindungen, die mindestens zwei reaktive Gruppen aufweisen, die unter geeigneten Bedingungen in der Lage sind, z.B. beim Erhitzen auf Temperaturen von mindestens 70°C, mit den Säuregruppen des schaumförmigen Hydrogel zu reagieren. In diesem Falle ist es auch möglich, gesteuert über die Eindringtiefe des Vernetzers, eine Modifikation der inhomogenen Vernetzungsdichte zu erreichen. Geeignete Vernetzer bilden mit den Carboxylgruppen der Polymermatrix kovalente oder ionische Bindungen. Geeignete Vernetzungsmittel sind Verbindungen, die mindestens zwei funktionelle Gruppen der gleichen oder unterschiedlicher Art aufweisen, z.B. Hydroxy-, Amino-, quaternäre Ammonium-, Isocyanato-, Epoxi-, Aziridino-, Ester- oder Amidgruppen. Bevorzugte Nachvernetzungsmittel sind neben Polyalkohole wie Glycerin, Butylenglykol, Propylenglykol auch Bisepoxide oder funktionalisierte Silane. Mit solchen Vernetzern kann die Reaktion z.B. in den Temperaturbereich von 10 - 170, vorzugsweise bei 20 - 160°C erfolgen. Das Auftragen der Vernetzungsmittel auf das geschäumte Material kann beispielsweise durch Sprühen, Tauchen oder durch Gasphasenabscheidung erfolgen.

Erfindungsgemäß kann man Schaum mit einem geringeren Neutralisationsgrad, typischerweise zwischen 0 und 60 %, bevorzugt 15 bis 40 %, als endgültig beabsichtigt herstellen und mindestens ein Alkanolamin nachträglich zur Neutralisation der Säuregruppen der superabsorbierenden Polymeren aufbringen z.B. durch Aufsprühen der Alkanolamine, deren Lösungen in Lösemitteln oder Lösemittelgemischen. Als Lösemittel für Alkanolamine können z.B. Verwendung finden: Wasser, Methanol, Ethanol, iso-Propanol und Aceton. Bevorzugt ist Wasser. Die Nachneutralisation erfolgt zweckmäßigerweise nach der Polymerisation und vor der Trocknung. Es ist aber auch möglich, mindestens ein Alkanolamin zu einem späteren Zeitpunkt im Prozeßablauf auf das schaumförmige Hydrogel aufzubringen.

Für die Anwendung von sekundären bzw. primären Alkanolaminen ist diese Vorgehensweise zwingend. Tertiäre Alkanolamine können - wie oben bereits beschrieben - zur Neutralisation der Monomeren (a) eingesetzt und außerdem - ebenso wie primäre, sekundäre und quaternäre Alkanolamine - zur Neutralisation der Säuregruppen des schaumförmigen Hydrogels nach dem Polymerisieren verwendet werden. In manchen Fällen hat sich eine Arbeitsweise als vorteilhaft herausgestellt, bei der man die Säuregruppen enthaltenden Monomeren (a) zunächst partiell mit einem tertiären Alkanolamin neutralisiert (z.B. zu 20 bis 50 Mol-%), dann polymerisiert und im Anschluß daran die restlichen freien Säuregruppen des schaumförmigen Hydrogels mit einem Alkanolamin, vorzugsweise einem primären Alkanolamin wie Ethanolamin, neutralisiert, wobei der Gesamtneutralisationsgrad der Säuregruppen im Hydrogel 55 bis 95, vorzugsweise 65 bis 85 Mol-% beträgt.

Die verwendeten. Alkanolamine können primär, sekundär, tertiär oder quaternär sein und einwertige, mehrwertige oder polyfunktionelle Basen darstellen. Die Alkanolamine können zusätzlich zu ihren Amino- und Hydroxylgruppen weitere funktionelle Gruppen wie z.B. Ester-, Urethan-, Ether-, Thioether- und Harnstoffgruppen tragen. Eingesetzt werden können z.B. niedermolekulare Verbindungen wie Triethanolamin, Methyldiethanolamin, Dimethylethanolamin, Ethanolamin, N-Hydroxyethylmorpholin, Dimethylaminodiglycol, N,N,N',N'-Tetra-(hydroxyethyl)-ethylendiamin, N,N,N' ,N'-Tetra-(hydroxypropyl)-ethylendiamin, Dimethylaminotriglycol, Diethylaminoethanol, 3-Dimethylamino-1,2-propandiol, Triisopropanolamin, Diisopropylaminoethanol, Cholinhydroxid, Cholincarbonat, 2-tert.-Butylaminoethanol, Tris(oxymethyl)aminomethan, 3-Amino-1-propanol, Isopropanolamin, 2-(2-Aminoethoxy)ethanol, 2-Amino-2-methyl-1-propanol oder aber Oligomere oder Polymere wie z.B. mit Ethylenoxid, Propylenoxid, Glycidol oder anderen Epoxiden umgesetzte Aminogruppen-tragende Polymerisate oder Kondensate wie z.B. Polyethylenimine oder Polyvinylamine, Umsetzungsprodukte aus mindestens bifunktionellen, niedermolekularen Alkanolaminen mit mindestens bifunktionellen Reagenzien, die in der Lage sind, entweder mit der Hydroxyl- oder der Aminogruppe der Alkanolamine zu reagieren, wie z.B. Carbonsäuren, Estern, Epoxiden, Isocyanaten.

Vorzugsweise kommen Triethanolamin, Methyldiethanolamin, Dimethylaminodiglycol, Dimethylethanolamin, Ethanolamin und/oder N,N,N' ,N' -Tetra-(hydroxyethyl)-ethylendiamin in Betracht.

Die erhaltenen Alkanolamin-haltigen Schäume sind klebrig. Die Klebrigkeit kann durch Puderung mit feinteiligen Pulvern vollständig beseitigt werden. Im Prinzip sind alle organischen oder anorganische Materialien in feiner Pulverform geeignet, sofern sie hydrophil sind, wie z.B. feinteiliges Siliziumoxid (Aerosil®), Silikate, Talkum, Guarkernmehl, Tarakernmehl, Johannisbrotkernmehl, alle Arten von Stärken, vernetzte oder nicht vernetzte Polyacrylsäuren oder deren Salze, Polyvinylalkohole, Copolymere der Maleinsäure, Titandioxid, Zeolithe, Cellulose, Carboxymethylcellulose und Hydroxyethylcellulose. Bevorzugt sind nicht wasserlösliche Materialien, insbesondere Talkum und Aerosil®. Die Puderung erfolgt zweckmäßigerweise nach der Polymerisation. Die Aufwandmengen liegen z.B. zwischen 0,01 und 10 %, bevorzugt zwischen 0,1 und 5 %, bezogen auf das Gewicht des Schaums.

Die erfindungsgemäßen Superabsorberschäume haben beispielsweise eine Dichte von $10^{-3}$ bis 0,9, vorzugsweise 0,05 bis 0,5 g/cm$^3$.

Bestimmung der Monomerschaumdichte:

Genau 100ml des Monomerschaums werden in einen Meßzylinder eingefüllt und das Gewicht dieses Schaumvolumens bestimmt. Durch Division des ermittelten Gewichts in g durch 100 wird die Dichte in g/cm$^3$ erhalten.

Bestimmung der Polymerschaumdichte:

Die Dichte von Superabsorberschäumen wird gravimetrisch bestimmt. Aus einer gleichmäßigen Schaumschicht mit einer definierten Dicke zwischen 3 und 5 mm schneidet man beispielsweise mit einem scharfen Messer Quadrate mit einer Seitenlänge von 5 cm aus. Diese Proben werden gewogen und das erhaltene Gewicht durch das aus den Maßen errechnete Volumen dividiert.

Bestimmung der Aufnahmekapazität:

Die Aufnahmekapazität des schaumförmigen Superabsorbers an Wasser pro Gramm Superabsorber wird an Schaumstücken bestimmt, die eine Dicke von 3 mm haben und jeweils 1 g wiegen. Die Prüfung der Aufnahmekapazität erfolgt hierbei nach dem sogenannten Teebeuteltest. Als Flüssigkeit dient dabei eine 0,9 %ige Kochsalzlösung. 1 g des schaumförmigen Materials wird in einen Teebeutel gefüllt, der dann verschlossen wird. Dabei ist darauf zu achten, daß der Teebeutel genügend Raum zum vollständigen Ausquellen bietet. Der Teebeutel wird danach eine bestimmte Zeit, z.B. 30 min, lang in die Flüssigkeit eingetaucht und nach einer Abtropfdauer von z.B. 10 Minuten zurückgewogen. Für die Bestimmung des Blindwertes wird ein Teebeutel ohne schaumförmigen Superabsorber in die Lösung eingetaucht und das Gewicht des Teebeutels unter den oben geschilderten Bedingungen bestimmt. Die Aufnahmekapazität ergibt sich dann aus folgender Gleichung (1):

$$\text{Aufnahmekapazität} = \frac{G_{TS} - G_T}{G_S} \tag{1},$$

wobei

$G_{TS}$ Gewicht des Teebeutels mit Superabsorberschaum
$G_T$ Gewicht des Teebeutels im Blindversuch
$G_S$ Gewicht des eingewogenen Superabsorberschaums

Bestimmung der Aufnahmegeschwindigkeit:

Die freie Aufnahmegeschwindigkeit (im folgenden mit FAG bezeichnet) wird dadurch ermittelt, daß man aus gleichmäßig 3 mm dicken Schaumschichten rechteckige Proben mit einem Gewicht von 1 g mit Hilfe eines scharfen Messers ausschneidet. Diese Proben werden in einer Petrischale mit 20 g 0,9 %iger Kochsalzlösung übergossen. Mit Hilfe einer Stoppuhr wird die Zeit ermittelt, die die Schaumprobe benötigt, um die 0,9%ige Kochsalzlösung vollständig aufzunehmen. Die Aufnahmegeschwindigkeit (FAG) in g/g-sec errechnet sich aus folgender Gleichung (2):

$$\text{FAG} = 20\ g/[1\ g \cdot \text{gemessene Zeit in sec}] \tag{2}$$

Tropfenaufnahmegeschwindigkeit (im folgenden mit TAG bezeichnet):

Zur Bestimmung der TAG wird auf die Oberfläche der Schaumschicht ein Tropfen einer 0,9 %igen Kochsalzlösung aufgesetzt und die Zeit gestoppt, bis der Tropfen vollständig in die Schaumschicht aufgenommen wurde. Der gleiche vorgang wird für die 2. Seite der Schaumschicht wiederholt und die beiden Werte mit oben bzw. unten gekennzeichnet.

Vertical Wicking Time (im folgenden mit VWT bezeichnet):

In eine Petrischale (10 cm Durchmesser, 1 cm hoch) wird 0.9%ige Kochsalzlösung bis zu einer Höhe von 0,5 cm eingefüllt. Anschließend wird ein Glasrohr (Durchmesser 1 cm, 15 cm hoch) knapp über dem Boden der Schale plaziert. Ein 6 cm langer Schaumstreifen mit quadratischer Grundfläche (5 x 5 mm) wird bei 2,4 und 6 cm mit einer Markierung versehen und innerhalb des Glasrohres in die Flüssigkeit gestellt. Gleichzeitig wird die Zeitmessung gestartet. Die Zeit in Sekunden, die zum Erreichen der jeweiligen Markierung nötig ist, wird bestimmt.

Die oben beschriebenen wasserabsorbierenden, schaumförmigen, vernetzten Polymerisate können für sämtliche Zwecke verwendet werden, für die die in der Literatur beschriebenen schaumförmigen Superabsorber eingesetzt werden. Sie werden z.B. in Sanitärartikeln, die zur Adsorption von Körperflüssigkeiten eingesetzt werden und in Verbandmaterial zur Abdeckung von Wunden verwendet. Sie eignen sich beispielsweise als wasserabsorbierender Bestandteil in Windeln, Damenbinden und Inkontinenzartikeln. Sie können in Form von Verbundmaterialien eingesetzt werden. Schaumförmige Superabsorber können außerdem als Dichtungsmaterial, als Bodenverbesserungsmittel, als Bodenersatzstoff und als Verpackungsmaterial verwendet werden. Spezielle Ausgestaltungen von Gegenständen, die schaumförmige Superabsorber enthalten, werden beispielsweise ausführlich in der WO-A-94/22502 beschrieben.

Die oben beschriebenen Schäume können aufgrund ihrer Eigenschaften verschiedene Funktionen in Hygieneartikeln bei der Speicherung von Körperflüssigkeiten erfüllen:

- Akquisition
- Distribution und/oder
- Speicherung

Die einzelnen Funktionen können entweder vollständig übernommen oder durch weitere Bestandteile unterstützt werden, so kann z: B. die Speicherung durch den Zusatz von Superabsorbergranulat erhöht werden oder die Akquisition und Distribution durch weitere Bestandteile wie high loft-Nonwovens, Polypropylen-Vliese. Polyester-Vliese oder chemisch modifizierte Zellstoffe optimiert werden.

Die Prozentangaben in den Beispielen bedeuten Gewichtsprozent, sofern aus dem Zusammenhang nichts anderes hervorgeht.

Beispiel 1

In einem Becherglas wurden mit Hilfe eines Magnetrührers die folgenden Komponenten vermischt.

| | |
|---|---|
| 348,55 g | Acrylsäure (4,84 mol) |
| 135,51 g | einer 37,3 %igen Natriumacrylatlösung in Wasser (0,54 mol) |
| 28,00 g | Polyethylenglykoldiacrylat von Polyethylenglykol der Molmasse 400 |
| 21,33 g | einer 15 %igen, wässrigen Lösung eines Additionsprodukts von 80 Mol Ethylenoxid an 1 mol eines linearen, gesättigten $C_{16}C_{18}$ Fettalkohols |
| 65,70 g | Wasser |

Zu dieser Lösung wurden unter Eiskühlung 400,90 g (2,69 mol) Triethanolamin so zugegeben, daß die Innentemperatur nicht über 16°C anstieg. Die erhaltene Lösung wurde in einen Druckbehälter überführt und dort für 25 min bei einem Druck von 12 bar mit Kohlendioxid gesättigt. Unter Druck wurden 26,67 g einer 3 %igen, wässrigen Lösung von 2,2'-Azobis(2-amidinopropan)dihydrochlorid zugegeben und mit einem starken Kohlendioxidstrom homogen untergemischt. Anschließend wurde für weitere 5 min Kohlendioxid durch die Reaktionsmischung geleitet. Die gesättigte Reaktionsmischung wurde bei einem Druck von 12 bar durch eine Düse mit einem Durchmesser von 1 mm ausgepreßt, wobei sich ein feinzelliger, gut fließfähiger Schaum bildete.

Der erhaltene Monomerschaum wurde auf eine DIN-A3 große Glasplatte mit 3 mm hohen Rändern aufgebracht und mit einer zweiten Glasplatte bedeckt. Die Schaumprobe wurde synchron von beiden Seiten mit zwei UV/VIS-Strahler (UV 1000 der Firma Höhnle) für 4 Minuten bestrahlt.

Die erhaltene Schaumschicht wurde in einem Vakuumtrockenschrank bei 70°C vollständig getrocknet. Zur Bestimmung der Eigenschaften wurde ein Teil des Schaums anschließend durch Besprühen mit Wasser auf eine Feuchte von 10 % eingestellt.

| | |
|---|---|
| Feststoffgehalt der Reaktionsmischung: | 81,04 % |
| Neutralisationsgrad: | 60 mol% |
| Monomerschaumdichte: | 0,18 $g/cm^3$ |
| Polymerschaumdichte: | 0,19 $g/cm^3$ |
| Schaumstruktur | homogen, vollständig offenzellig, keine Hautbildung |

Beispiel 2

In einem Becherglas wurden mit Hilfe eines Magnetrührers die folgenden Komponenten vermischt:

| | |
|---|---|
| 303,24 g | Acrylsäure (4,21 mol) |
| 117,90 g | einer 37,3 %igen Natriumacrylatlösung in Wasser (0,47 mol) |
| 24,36 g | Polyethylenglykoldiacrylat von Polyethylenglykol der Molmasse 500 |
| 5,57 g | Additionsprodukt von 80 Mol Ethylenoxid an 1 mol eines linearen, gesättigten $C_{16}C_{18}$-Fettalkohols |
| 0,15 g | Wasser |

Zu dieser Lösung wurden unter Eiskühlung 348,79 g (2,69 mol) Triethanolamin so zugegeben, daß die Innentemperatur nicht über 16°C anstieg. Die erhaltene Lösung wurde in einem Druckbehälter überführt und dort für 25 min bei einem Druck von 12 bar mit Kohlendioxid gesättigt. Unter Druck wurden 13,92 g einer 3 %igen, wässrigen Lösung von 2,2'-Azobis(2-amidinopropan)dihydrochlorid zugegeben und mit einem starken Kohlendioxidstrom homogen untergemischt. Anschließend wurde für weitere 5 min Kohlendioxid durch die Reaktionsmischung geleitet. Die gesättigte Reaktionsmischung wurde bei einem Druck von 12 bar durch eine Düse mit einem Durchmesser von 1 mm ausgepreßt, wobei sich ein feinzelliger, fließfähiger Schaum bildet.

Der erhaltene Monomerschaum wurde auf eine DIN-A3 große Glasplatte mit 3 mm hohen Rändern aufgebracht und mit einer zweiten Glasplatte bedeckt. Die Schaumprobe wurde synchron von beiden Seiten mit zwei UV/VIS-Strahler (UV 1000 der Firma Höhnle) für 4 Minuten bestrahlt.

Die erhaltene Schaumschicht wurde auf beiden Seiten mit ca. 0,3 g Talkum bepudert und in einem Vakuumtrockenschrank bei 70°C vollständig getrocknet. Zur Bestimmung der Eigenschaften wurde ein Teil des Schaums anschließend durch Besprühen mit Wasser auf eine Feuchte von 10 % eingestellt.

| Festgehalt der Reaktionsmischung | 88,67 % |
| Neutralisationsgrad | 60 mol-% |
| Monomerschaumdichte | 0,19 g/cm$^3$ |
| Polymerschaumdichte | 0,17 g/cm$^3$ |
| Schaumstruktur | homogen, vollständig offenzel lig, keine Hautbildung |

Beispiel 3

In einem Becherglas wurden mit Hilfe eines Magnetrührers die folgenden Komponenten vermischt:

| 278,84 g | Acrylsäure (3,87 mol) |
| 108,41 g | einer 37,3 %igen Natriumacrylatlösung in Wasser (0,43 mol) |
| 22,40 g | Polyethylenglykoldiacrylat von Polyethylenglykol der Molmasse 500 |
| 17,07 g | einer 15 %igen, wäßrigen Lösung eines Additionsprodukts von 80 Mol Ethylendioxid an 1 mol eines linearen, gesättigten $C_{16}C_{18}$-Fettalkohols |
| 5,12 g | einer 25 %igen, wässrigen Lösung des Salzes aus Diethanolamin und Heptadecafluorooctansulfonsäure |
| 52,56 g | Wasser |

Zu dieser Lösung wurden unter Eiskühlung 320,72 g (2,15 mol) Triethanolamin so zugegeben, daß die Innentemperatur nicht über 16°C anstieg. Die erhaltene homogene Mischung wurde in einen Druckbehälter überführt und dort für 25 min bei einem Druck von 10 bar mit Kohlendioxid gesättigt. Unter Druck wurden 21,33 g einer 3 %igen, wässrigen Lösung von 2,2'-Azobis(2-amidinopropan)dihydrochlorid zugegeben und mit einem starken Kohlendioxidstrom homogen untergemischt. Anschließend wurde für weitere 5 min Kohlendioxid durch die Reaktionsmischung geleitet. Die gesättigte Reaktionsmischung wurde bei einem Druck von 12 bar durch eine Düse mit einem Durchmesser von 1 mm ausgepreßt, wobei sich ein feinzelliger, gut fließfähiger Schaum bildete.

Der erhaltene Monomerschaum wurde auf eine DIN-A3 große Glasplatte mit 3 mm hohen Rändern aufgebracht und mit einer zweiten Glasplatte bedeckt. Die Schaumprobe wurde synchron von beiden Seiten mit zwei UV/VIS-Strahler (UV 1000 der Firma Höhnle) für 4 Minuten bestrahlt.

Die erhaltene Schaumschicht wurde auf beiden Seiten mit ca. 0,3 g Talkum bepudert und in einem Vakuumtrockenschrank bei 70°C vollständig getrocknet. Zur Bestimmung der Eigenschaften wurde ein Teil des Schaums anschließend durch Besprühen mit Wasser auf eine Feuchte von 10 % eingestellt.

| Festgehalt der Reaktionsmischung | 81,0 % |
| Neutralisationsgrad | 60 mol-% |
| Monomerschaumdichte | 0,18 g/cm$^3$ |
| Polymerschaumdichte | 0,18 g/cm$^3$ |
| Schaumstruktur | homogen, vollständig offen zellig, keine Hautbildung |

Vergleichsbeispiel 1

In einem verschlossenen Glas mit Schraubverschluß wurden mit Hilfe eines Magnetrührers die folgenden Komponenten vermischt:

| 37,64 g | Acrylsäure (0,52 mol) |
| 395,08 g | einer 37,3 %igen Natriumacrylatlösung in Wasser (1,57 mol) |
| 9,25 g | Guarkernmehl |
| 1,85 g | Polyethylenglykoldiacrylat eines Polyethylenglykols der Molmasse 400 |
| 58,58 g | einer 15 %igen, wässrigen Lösung eines Additionsproduktes von 80 Mol Ethylenoxid an 1 mol eines linearen, gesättigten $C_{16}C_{18}$-Fettalkohols |
| 6,85 g | Wasser |

Die erhaltene homogene Mischung wurde in einen geschlossenen 2 1-Kolben mit Kühlmantel eingefüllt, in den von unten her Kohlendioxid eingeleitet wurde. In den Kolben waren zwei Schneebesen der Firma BOKU eingesetzt, die über ein Getriebe mit einem Rührer RW28 W der Firma IKA verbunden waren. Der Kohlendioxidstrom wurde so eingestellt, daß er mit einer Geschwindigkeit von 100 l/h durch die Reaktionsmischung perlte. Der Rührmotor wurde zunächst auf eine Drehzahl von 200 UpM eingestellt und für 20 min Kohlendioxid durch die Mischung geleitet um gelösten Sauerstoff zu entfernen. Während dieser Zeit wurde die Innentemperatur mit Hilfe des Kühlmantels und eines Thermostaten auf 16°C eingestellt. Anschließend wurden 4,63 g Pentan und 20,97 g einer 3 %igen Lösung von 2,2'-Azobis(2-amidinopropan)dihydrochlorid in Wasser zugegeben und die Rührdrehzahl auf 735 UpM eingestellt. Die Mischung wurde bei dieser Drehzahl für 3,5 min aufgeschlagen. Nach Ende der Aufschlagsperiode wurde ein feinzelliger, gut fließfähiger Schlagschaum erhalten.

Der Monomerschaum wurde auf eine DIN-A3 große Glasplatte mit 3 mm hohen Rändern aufgebracht und mit einer zweiten Glasplatte bedeckt. Die Schaumprobe wurde synchron von beiden Seiten mit zwei UV/VIS-Strahler (UV 1000 der Firma Höhnle) für 4 Minuten bestrahlt.

Die erhaltene Schaumschicht wurde in einem Vakuumtrockenschrank bei 70°C vollständig getrocknet und anschließend durch Besprühen mit Wasser auf einen Feuchtgehalt von 25 % eingestellt.

| | |
|---|---|
| Feststoffgehalt der Reaktionsmischung | 38,4 % |
| Neutralisationsgrad | 75 mol-% |
| Monomerschaumdichte | 0,31 g/cm$^3$ |
| Polymerschaumdichte | 0,32 g/cm$^3$ |
| Schaumstruktur | homogen, vollständig offenzellig, keine Hautbildung |
| Griff | feucht, kaum klebrig |

Vergleichsbeispiel 2

In einem verschlossenen Glas mit Schraubverschluß wurden mit Hilfe eines Magnetrührers die folgenden Komponenten vermischt:

| | |
|---|---|
| 127,93 g | Acrylsäure (1,67 mol) |
| 93,43 g | einer 37,3 %igen Natriumacrylatlösung in Wasser (0,42 mol) |
| 12,60 g | Guarkernmehl |
| 6,29 g | Polyethylenglykoldiacrylat eines Polyethylenglykols der Molmasse 400 |
| 58,78 g | einer 15 %igen, wässrigen Lösung eines Additionsprodukts von 80 Mol Ethylenoxid an 1 mol eines linearen, gesättigten $C_{16}C_{18}$-Fettalkohols |
| 63,02 g | Wasser |

Die erhaltene homogene Mischung wurde in einen geschlossenen 2 1-Kolben mit Kühlmantel und Tropftrichter eingefüllt, in den von unten Kohlendioxid eingeleitet wurde. In den Kolben waren zwei Schneebesen der Firma BOKU eingesetzt, die über ein Getriebe mit einem Rührer RW28W der Firma IKA verbunden waren. Der Kohlendioxidstrom wurde so eingestellt, daß er mit einer Geschwindigkeit von 100 l/h durch die Reaktionsmischung perlte. Der Rührmotor wurde zunächst auf eine Drehzahl von 200 UpM eingestellt und für 20 min Kohlendioxid durch die Mischung geleitet um gelösten Sauerstoff zu entfernen. Während dieser Zeit wurden 152,42 g Triethanolamin (1,04 mol) unter KÜhlen so zugetropft, daß eine Endtemperatur von 16°C erreicht wurde.

Anschließend wurden 4,63 g Pentan und 20,99 g einer 3 %igen Lösung von 2,2'-Azobis(2-amidinopropan)dihydrochlorid in Wasser zugegeben und die Rührerdrehzahl auf 735 UpM erhöht. Die Mischung wurde bei dieser Drehzahl für 3,5 min aufgeschlagen. Nach Ende der Aufschlagsperiode wurde ein feinzelliger, gut fließfähiger Schlagschaum erhalten.

Der erhaltene Monomerschaum wurde auf eine DIN-A3 große Glasplatte mit 3 mm hohen Rändern aufgebracht und mit einer zweiten Glasplatte bedeckt. Die Schaumprobe wurde synchron von beiden Seiten mit zwei UV/VIS-Strahler (UV 1000 der Firma Höhnle) für 4 Minuten bestrahlt.

Die erhaltene Schaumschicht wurde auf beiden Seiten mit ca. 0,3 g Talkum bepudert und in einem Vakuumtrockenschrank bei 70°C vollständig getrocknet. Zur Bestimmung der Eigenschaften wurde ein Teil des Schaums anschließend durch Besprühen mit Wasser auf eine Feuchte von 10 % eingestellt.

| | |
|---|---|
| Festgehalt der Reaktionsmischung | 63,6 % |
| Neutralisationsgrad: | 70 mol% |

(fortgesetzt)

| | |
|---|---|
| Monomerschaumdichte: | 0,26 g/cm$^3$ |
| Polymerschaumdichte: | 0,26 g/cm$^3$ |
| Schaumstruktur | homogen, vollständig offenzellig, keine Hautbildung |

Vergleichsbeispiel 3 (entsprechend Beispiel 1 der JP-A-08073507)

In einem geschlossenen Kolben mit Rührer und Stickstoffeinleitung von unten wurde aus den folgenden Komponenten eine Mischung hergestellt:

| | |
|---|---|
| 180,00 g | Acrylsäure (2,50 mol) |
| 186,50 g | Triethanolamin (1.25 mol) |
| 86,18 g | Wasser |
| 4,60 g | hydroxyethylcellulose |
| 4,00 g | Trimethylolpropantriacrylat |
| 3,33 g | einer 15 %igen, wässrigen Natriumperoxodisulfatlösung |
| 2,50 g | einer 0,5 %igen Lösung von Ascorbinsäure in Wasser |

Die erhaltene Mischung wurde zwischen zwei Teflonplatten gefüllt, die durch eine Gummidichtung auf 1 mm Abstand gehalten wurden und in einem Umlufttrockenschrank bei 80°C auspolymerisiert.
Die erhaltene Gelschicht mit einer Schichtdicke von 0,9 mm hatte einen Festgehalt von 90% und war stark klebrig.

Vergleichsbeispiel 4 (entsprechend Beispiel 9 der WO-A-95/02002)

| | |
|---|---|
| 641,56 g | einer 40,0 %igen Kaliumacrylatlösung in Wasser (2,33 mol) |
| 235,00 g | Wasser |
| 72,06 g | Acrylsäure (1,00 mol) |
| 0,99 g | Triallylamin |
| 3,29 g | Polyvinylalkohol |
| 0,82 g | 2,2'-Azobis(2-amidinopropan)dihydrochlorid |

Aus den vorstehenden Komponenten wurde eine Reaktionsmischung in einem Becherglas hergestell und durch Zugabe von Trockeneis mit $CO_2$ gesättigt. Die erreichte Endtemperatur betrug 4°C. Die Mischung wurde in eine gekühlt Porzellanschale in einer Schichtdicke von 1 cm gefüllt und zur Polymerisation von oben mit einem UV-Strahler (UV 1000 der Firma Höhnle) bestrahlt.
Die erhaltene Schaumschicht wurde in einem Vakuumtrockenschrank bei 85°C vollständig getrocknet und anschließend durch Besprühen mit Wasser auf einen Feuchtegehalt von 25 % eingestellt.

| | |
|---|---|
| Feststoffgehalt der Reaktionsmischung | 35,00 % |
| Neutralisationsgrad: | 70,00 mol% |
| Monomerschaumdichte: | nicht bestimmbar |
| Polymerschaumdichte: | 0,60 g/cm$^3$ |
| Schaumstruktur | homogen, vollständig geschlossenzellig, beidseitig starke Hautbildung |

Vergleichsbeispiel 5 (entsprechend Beispiel 9 der WO-A-95/02002)

Der Schaum aus Vergleichsbeispiel 4 wurde zerkleinert, im Vakuumtrockenschrank bei 85°C getrocknet, gemahlen und die Fraktion mit einer Partikelgröße zwischen 90 und 850 µm abgetrennt.
Diese Pulver wurden mit einer Mischung von 0,5% 1,3-Dioxolan-2-on, 2,0% Wasser und 2,0% Ethanol, jeweils bezogen auf das Pulver, besprüht und 30 min auf 200°C erhitzt. Die Eigenschaften dieses SAP sind in den Tabellen angegeben.

Vergleichsbeispiel 6 (entsprechend Beispiel 2 der US-A-5,750,585)

| 6,00 g | Wasser |
|---|---|
| 1,98 g | Acrylsäure (1,00 mol) |
| 0,02 g | N,N'-Methylenbisacrylamid |
| 0,50 g | einer 10 %igen, wässrigen Lösung von N,N,N',N'-Tetramethylethylendiamin |

Aus den angegebenen Bestandteilen wurde eine Lösung hergestellt und in ein Reagenzglas mit 30 mm Durchmesser gefüllt, anschlie-Bend wurden 0,5 ml einer 10%igen, wässrigen Lösung von Ammoniumpersulfat zugegeben und durch Schüttel untergemischt. Die Lösung wurde durch Eintauchen in ein Wasserbad auf 60°C erwärmt und anschließend aus dem Wasserbad genommen. Dann wurden lml einer 70%igen Suspension von $NaHCO_3$ in Wasser tropfenweise zugegeben, wobei das Reagenzglas geschüttelt wurde. Der erhaltene Schaum wurde aus dem Reagenzglas entnommen, vollständig getrocknet und anschließend durch Besprühen mit Wasser auf einen Feuchtegehalt von 25% eingestellt.

Weitere anwendungstechnische Eigenschaften der in den voranstehenden Beispielen und Vergleichsbeispielen beschriebenen Polymeren sind in den nachfolgenden Tabellen zusammengestellt. Sie zeigen, daß die erfindungsgemäßen Produkte signifikant verbesserte Aufnahmeverhalten und Verteilungswirkung aufweisen.

Tabelle 1:

| Aufnahmekapazitäten | | | |
|---|---|---|---|
| Beispiel | Aufnahmekapazität [g/g] | Feuchtgehalt [%] | Aufnahmekapazität bei 100 % Festgehalt [g/g] |
| 1 | 58,3 | 10 | 64,8 |
| 2 | 57,9 | 10 | 64,3 |
| 3 | 53,8 | 25 | 71,1 |
| Vergleichsbeispiel 1 | 36,2 | 25 | 48,3 |
| Vergleichsbeispiel 2 | 55,9 | 10 | 62,1 |
| Vergleichsbeispiel 3 | 14,3[a)]/24,3[b)] | 10 | 18,8[a)]/27,0[b)] |
| Vergleichsbeispiel 4 | 7,5 | 25 | 10,0 |
| Vergleichsbeispiel 5 | 45,3 | <1 | 60,4 |
| Vergleichsbeispiel 6 | 25,6 | 25 | 34,1 |

a) Die Aufnahmewerte wurden nach einer Quellzeit von 60 min ermittelt

b) Die Aufnahmewerte wurden nach einer Quellzeit von 18 h ermittelt

Tabelle 2:

| Aufnahmeverhalten anhand Aufnahmegeschwindigkeiten | | |
|---|---|---|
| Beispiel | FAG [g/g sec] | TAG unten/oben [sec] |
| 1 | 10,00 | <1/<1 |
| 2 | 6,67 | <1/<1 |
| 3 | 4,00 | <1/<1 |
| Vergleichsbeispiel 1 | 0,79 | 9/11 |
| Vergleichsbeispiel 2 | 3,25 | 2/2 |
| Verlgeichsbeispiel 3 | <0,02 | >100/>100 |
| Vergleichsbeispiel 4 | <0,02 | >100/>100 |

**EP 1 165 673 B1**

Tabelle 2:   (fortgesetzt)

| Aufnahmeverhalten anhand Aufnahmegeschwindigkeiten | | |
|---|---|---|
| Beispiel | FAG [g/g sec] | TAG unten/oben [sec] |
| Vergleichsbeispiel 5 | 0,52 | a) |
| Vergleichsbeispiel 6 | <0,05 | 14/35 |

a) kann an einem Pulver nicht ermittelt werden

Tabelle 3:

| Verteilungswirkung anhand von Wickingverhalten | | | |
|---|---|---|---|
| | Vertical Wicking | | |
| Beispiel | 2 cm [sec] | 4 cm [sec] | 6 cm [sec] |
| 1 | 8 | 23 | 48 |
| 2 | 7 | 24 | 46 |
| 3 | 10 | 32 | 68 |
| Vergleichsbeispiel 1 | 128 | 600 | >600 |
| Vergleichsbeispiel 2 | 12 | 77 | 162 |
| Vergleichsbeispiel 3 | >600 | >600 | >600 |
| Vergleichsbeispiel 4 | 215 | >600 | >600 |
| Vergleichsbeispiel 5 | a) | a) | a) |
| Vergleichsbeispiel 6 | 228 | >600 . | >600 |

a) kann an einem Pulver nicht ermittelt werden

**Patentansprüche**

1.  Wasserabsorbierende, schaumförmige, vernetzte Polymerisate, die eine freie Aufnahmegeschwindigkeit (FAG) für eine 0,9 gew.-%ige wäßrige Kochsalzlösung von 4,0 bis 100 g/g sec. haben und die erhältlich sind durch

    (I) Schäumen einer polymerisierbaren wäßrigen Mischung, die

        (a) Säuregruppen enthaltende monoethylenisch ungesättigte Monomere oder mit Alkalimetallbasen, Ammoniak oder Aminen neutralisierte Säuregruppen enthaltende monoethylenisch ungesättigte Monomere.
        (c) Vernetzer,
        (d) Initiatoren und
        (e) 0,1 bis 20 Gew.-% mindestens eines Tensids

    enthält, wobei man zum Schäumen ein gegenüber Radikalen inertes Gas unter einem Druck von 2 bis 400 bar in der polymerisierbaren wäßrigen Mischung löst und sie anschließend auf Atmosphärendruck entspannt und

    (II) Polymerisieren der geschäumten Mischung unter Bildung eines schaumförmigen Hydrogels.

2.  Wasserabsorbierende, schaumförmige, vernetzte Polymerisate nach Anspruch 1, **dadurch gekennzeichnet, daß** sie erhältlich sind durch

    (I) Schäumen einer polymerisierbaren wäßrigen Mischung, die

20

(a) zu 15 bis 90 Mol-% mit Alkalimetallbasen, Ammoniak oder Aminen neutralisierte Säuregruppen enthaltende monoethylenisch ungesättigte Monomere,

(b) andere monoethylenisch ungesättigte Monomere,

(c) Vernetzer,

(d) Initiatoren,

(e) 0,1 bis 20 Gew.-% mindestens eines Tensids

(f) mindestens einen Lösevermittler und

(g) Verdicker, Schaumstabilisatoren, Polymerisationsregler, Füllstoffe und/oder Zellkeimbildner

enthält, wobei man zum Schäumen ein gegenüber Radikalen inertes Gas unter einem Druck von 2 bis 400 bar in der polymerisierbaren wäßrigen Mischung löst und sie anschließend auf Atmosphärendruck entspannt und

(II) Polymerisieren der geschäumten Mischung unter Bildung eines schaumförmigen Hydrogels und Einstellen des Wassergehalts des schaumförmigen Polymerisats auf 1 bis 60 Gew.-%.

3. Wasserabsorbierende, schaumförmige, vernetzte Polymerisate nach Anspruch 1 oder 2, **dadurch gekennzeichnet, daß** sie erhältlich sind durch Neutralisation der Säuregruppen enthaltenden Monomeren (a) mit tertiären Alkanolaminen und/oder durch Neutralisation der freien Säuregruppen des schaumförmigen Hydrogels nach dem Polymerisieren mit mindestens einem Alkanolamin, wobei der Neutralisationsgrad jeweils mindestens 20 Mol-% beträgt.

4. Wasserabsorbierende, schaumförmige, vernetzte Polymerisate nach einem der Ansprüche 1 bis 3, **dadurch gekennzeichnet, daß** der Neutralisationsgrad mindestens 40 Mol-% beträgt.

5. Wasserabsorbierende, schaumförmige, vernetzte Polymerisate nach einem der Ansprüche 1 bis 4, **dadurch gekennzeichnet, daß** sie eine Vertikal Wicking Time (VWT = Zeit für die Ausbreitung einer 0,9 gew.-%igen wäßrigen Kochsalzlösung in einem Schaumstoff in vertikaler Richtung) für eine Höhe von 4 cm von 0,2 bis 120 Sekunden haben.

6. Verfahren zur Herstellung von wasserabsorbierenden, schaumförmigen, vernetzten Polymerisaten durch Schäumen einer polymerisierbaren Mischung aus

(a) Säuregruppen enthaltenden monoethylenisch ungesättigten Monomeren oder mit Alkalimetallbasen, Ammoniak oder Aminen neutralisierte Säuregruppen enthaltende monoethylenisch ungesättigte Monomere,

(c) Vernetzer,

(d) Initiatoren und

(e) 0,1 bis 20 Gew.-% mindestens eines Tensids

in einer ersten Verfahrensstufe mit einem gegenüber Radikalen inertem Gas und Polymerisieren des so erhaltenen Schaums in einer zweiten Verfahrensstufe unter Bildung eines schaumförmigen Hydrogels, **dadurch gekennzeichnet, daß** man in der ersten Verfahrensstufe ein gegenüber Radikalen inertes Gas unter einem Druck von 2 bis 400 bar in der polymerisierbaren wäßrigen Mischung löst und sie anschließend unter Schaumbildung auf Atmosphärendruck entspannt.

7. Verfahren nach Anspruch 6, **dadurch gekennzeichnet, daß** man die polymerisierbare wäßrige Mischung unter einem Druck von 5 bis 40 bar mit Kohlendioxid oder Stickstoff sättigt.

8. Verfahren nach Anspruch 6 oder 7, **dadurch gekennzeichnet, daß** man mindestens 20 Mol-% der Säuregruppen enthaltenden Monomeren (a) mit tertiären Alkanolaminen neutralisiert und/oder die freien Säuregruppen des schaumförmigen Hydrogels nach dem Polymerisieren zu mindestens 20 Mol-% mit mindestens einem Alkanolamin neutralisiert.

9. Verfahren nach Anspruch 6 oder 7, **dadurch gekennzeichnet, daß** man mindestens 40 Mol-% der Säuregruppen enthaltenden Monomeren (a) mit tertiären Alkanolaminen neutralisiert und/oder die freien Säuregruppen des schaumförmigen Hydrogels nach dem Polymerisieren zu mindestens 40 Mol-% mit mindestens einem Alkanolamin neutralisiert.

10. Verfahren nach einem der Ansprüche 6 bis 7, **dadurch gekennzeichnet, daß** man als Alkanolamine Triethanolamin, Ethanolamin und/oder N,N,N'N'-Tetra-(hydroxyethyl)-ethylendiamin einsetzt.

11. Verwendung der wasserabsorbierenden, schaumförmigen, vernetzten Polymerisate nach den Ansprüchen 1 bis 5 in Sanitärartikeln, die zur Absorption von Körperflüssigkeiten eingesetzt werden, in Verbandmaterial zur Abdeckkung von Wunden, als Dichtungsmaterial, als Bodenverbesserungsmittel, als Bodenersatzstoff und als Verpakkungsmaterial.

**Claims**

1. A water-absorbing, expanded, crosslinked polymer which has a free absorption speed (FAS) for a 0.9% by weight aqueous sodium chloride solution of from 4.0 to 100 g/g sec and which is obtainable by

   (I) foaming a polymerizable aqueous mixture which comprises

   (a) monoethylenically unsaturated monomers which contain acidic groups, or monoethylenically unsaturated monomers which contain acidic groups neutralized with alkali metal bases, ammonia or amines,
   (c) crosslinkers,
   (d) initiators and
   (e) 0.1-20% by weight of at least one surfactant,

   where the foaming takes place by dissolving a gas which is inert to free radicals under a pressure of 2-400 bar in the polymerizable aqueous mixture, and then decompressing the latter to atmospheric pressure, and

   (II) polymerizing the foamed mixture to form an expanded hydrogel.

2. A water-absorbing, expanded, crosslinked polymer as claimed in claim 1 and obtainable by

   (I) foaming a polymerizable aqueous mixture which comprises

   (a) monoethylenically unsaturated monomers which contain acidic groups 15-90 mol% neutralized with alkali metal bases, ammonia or amines,
   (b) other monoethylenically unsaturated monomers,
   (c) crosslinkers,
   (d) initiators,
   (e) 0.1-20% by weight of at least one surfactant,
   (f) at least one solubilizer and
   (g) thickeners, foam stabilizers, polymerization regulators, fillers and/or cell nucleating agents,

   where the foaming takes place by dissolving a gas which is inert to free radicals under a pressure of 2-400 bar in the polymerizable aqueous mixture, and then decompressing the latter to atmospheric pressure, and

   (II) polymerizing the foamed mixture to form an expanded hydrogel and, where appropriate, adjusting the water content of the expanded polymer to 1-60% by weight.

3. A water-absorbing, expanded, crosslinked polymer as claimed in claim 1 or 2, which is obtainable by neutralizing the monomers (a) which contain acidic groups using tertiary alkanolamines and/or by neutralizing the free acidic groups of the expanded hydrogel after the polymerization using at least one alkanolamine, the degree of neutralization being at least 20 mol% in each case.

4. A water-absorbing, expanded, crosslinked polymer as claimed in any of claims 1 to 3, wherein the degree of neutralization is at least 40 mol%.

5. A water-absorbing, expanded, crosslinked polymer as claimed in any of claims 1 to 4, which has a vertical wicking time (VWT = time for a 0.9% by weight aqueous sodium chloride solution to spread vertically in the foam) of 0.2-120 seconds for a height of 4 cm.

6. A process for producing water-absorbing, expanded, crosslinked polymers by foaming a polymerizable mixture of

(a) monoethylenically unsaturated monomers which contain acidic groups, or monoethylenically unsaturated monomers which contain acidic groups which are neutralized with alkali metal bases, ammonia or amines,
(c) crosslinkers,
(d) initiators,
(e) 0.1-20% by weight of at least one surfactant,

in a first stage with a gas which is inert to free radicals, and in a second stage polymerizing the foam obtained in this way to form an expanded hydrogel, wherein in the first stage a gas which is inert to free radicals is dissolved under a pressure of 2-400 bar in the polymerizable aqueous mixture, and then the latter is decompressed to atmospheric pressure to form a foam.

7. A process as claimed in claim 6, wherein the polymerizable aqueous mixture is saturated with carbon dioxide or nitrogen under a pressure of 5 - 40 bar.

8. A process as claimed in claim 6 or 7, wherein at least 20 mol% of the monomers (a) which contain acidic groups are neutralized with tertiary alkanolamines and/or the free acidic groups of the expanded hydrogel are at least 20 mol% neutralized with at least one alkanolamine after the polymerization.

9. A process as claimed in claim 6 or 7, wherein at least 40 mol% of the monomers (a) which contain acidic groups are neutralized with tertiary alkanolamines and/or the free acidic groups of the expanded hydrogel are at least 40 mol% neutralized with at least one alkanolamine after the polymerization.

10. A process as claimed in either of claims 6 and 7, wherein triethanolamine, ethanolamine and/or N,N,N'N'-tetra (hydroxyethyl)ethylenediamine are employed as alkanolamines.

11. The use of the water-absorbing, expanded, crosslinked polymers as claimed in claims 1 to 5 in hygiene articles employed to absorb body fluids, in dressing material for covering wounds, as sealing material, as soil improver, as soil substitute and as packaging material.

**Revendications**

1. Polymères hydrophiles réticulés sous forme de mousse présentant un taux d'absorption libre (VAL) comprise entre 4,0 g/g sec et 100 g/g sec pour une solution aqueuse de chlorure de sodium à 0,9% en poids, et que l'on peut obtenir au moyen

(I) d'une production de mousse à partir d'un mélange aqueux polymérisable contenant

(a) des monomères à insaturation monoéthylénique contenant des groupes acide ou des monomères à insaturation monoéthylénique contenant des groupes acide neutralisés à l'aide des bases de métal alcalin, de l'ammoniac ou des amines,
(c) des agents de réticulation,
(d) des amorceurs et
(e) au moins un agent tensio-actif à raison de 0,1 à 20% en poids,

en procédant, pour réaliser la production de mousse, à une dissolution d'un gaz qui est inerte envers les radicaux, dans le mélange aqueux polymérisable, à une pression comprise entre 2 bars et 400 bars, suivie d'une détente à la pression atmosphérique, et
(II) d'une polymérisation du mélange expansé afin d'obtenir un hydrogel sous forme de mousse.

2. Polymères hydrophiles réticulés sous forme de mousse selon la revendication 1, **caractérisés en ce que** l'on peut les obtenir au moyen

(I) d'une production de mousse à partir d'un mélange aqueux polymérisable contenant

(a) des monomères à insaturation monoéthylénique contenant des groupes acide neutralisés à l'aide des bases de métal alcalin, de l'ammoniac ou des aminés, à raison de 15% à 90% en poids,
(b) d'autres monomères à insaturation monoéthylénique,

(c) des agents de réticulation,

(d) des amorceurs,

(e) au moins un agent tensio-actif à raison de 0,1 à 20% en poids,

(f) au moins un auxiliaire de dissolution et

(g) des épaississants, des stabilisants de mousse, des régulateurs de polymérisation, des charges et/ou des agents d'amorçage, de germination de cellule,

en procédant, pour réaliser la production de mousse, à une dissolution d'un gaz qui est inerte envers les radicaux, dans le mélange aqueux polymérisable, à une pression comprise entre 2 bars et 400 bars, suivie d'une détente à la pression atmosphérique, et

(II) d'une polymérisation du mélange expansé afin d'obtenir un hydrogel sous forme de mousse, et d'une régulation de la teneur en eau du polymère sous forme de mousse, à une valeur dans la plage de 1% à 60% en poids.

3. Polymères hydrophiles réticulés sous forme de mousse selon la revendication 1 ou 2, **caractérisés en ce que** l'on peut les obtenir au moyen d'une neutralisation, à l'aide des alcanolamines tertiaires, des monomères (a) contenant les groupes acide et/ou au moyen d'une neutralisation des groupes acide libres de l'hydroge sous forme de mousse, ultérieurement à la polymérisation, à l'aide d'au moins une alcanolamine, le degré de neutralisation étant à chaque fois au moins de 20% en moles.

4. Polymères hydrophiles réticulés sous forme de mousse selon l'une quelconque des revendications 1 à 3, **caractérisés en ce que** le degré de neutralisation est au moins de 40% en moles.

5. Polymères hydrophiles réticulés sous forme de mousse selon l'une quelconque des revendications 1 à 4, **caractérisés en ce qu'**ils présentent une durée d'absorption capillaire verticale (Vertikal Wicking Time ; VWT = durée pour la diffusion verticale d'une solution aqueuse du chlorure de sodium à 0,9% en poids dans un produit alvéolaire) comprise entre 0,2 seconde et 120 secondes pour une hauteur de 4 cm.

6. Procédé pour la préparation des polymères hydrophiles réticulés sous forme de mousse, au moyen d'une production de mousses à partir d'un mélange aqueux polymérisable constitué de

(a) monomères à insaturation monoéthylénique contenant des groupes acide ou des monomères à insaturation monoéthylénique contenant des groupes acide neutralisés à l'aide des bases de métal alcalin, de l'ammoniac ou des amines,

(c) agents de réticulation,

(d) amorceurs et

(e) au moins un agent tensio-actif à raison de 0,1 à 20% en poids,

dans une première étape de procédé, avec un gaz inerte envers les radicaux, et d'une polymérisation de la mousse ainsi obtenue dans une deuxième étape du procédé, afin d'obtenir un hydrogel sous forme de mousse, **caractérisé en ce que** l'on procède, dans la première étape du procédé, à une dissolution d'un gaz qui est inerte envers les radicaux, dans le mélange aqueux polymérisable, à une pression comprise entre 2 bars et 400 bars, suivie d'une détente à la pression atmosphérique afin de réaliser une production de mousse.

7. Procédé selon la revendication 6, **caractérisé en ce que** l'on procède à une saturation du mélange aqueux polymérisable à l'aide du dioxyde de carbone ou de l'azote, à une pression comprise entre 5 bars et 40 bars.

8. Procédé selon la revendication 6 ou 7, **caractérisé en ce que** l'on neutralise, à l'aide des alcanolamines tertiaires, au moins 20% en moles des monomères (a) contenant des groupes acides et/ou **en ce que** l'on neutralise, à l'aide d'au moins une alcanolamine, au moins 20% en moles des groupes acide libres de l'hydrogel sous forme de mousse, ultérieurement à la polymérisation.

9. Procédé selon la revendication 6 ou 7, **caractérisé en ce que** l'on neutralise, à l'aide des alcanolamines tertiaires, au moins 40% en moles des monomères (a) contenant des groupes acides et/ou **en ce que** l'on neutralise, à l'aide d'au moins une alcanolamine, au moins 40% en moles des groupes acide libres de l'hydrogel sous forme de mousse, ultérieurement à la polymérisation.

10. Procédé selon l'une quelconque des revendications 6 ou 7, **caractérisé en ce que** l'on met en oeuvre, en tant qu'alcanolamines, la triéthanolamine, l'éthanolamine et/ou la N,N,N',N'-tétra-(hydroxyéthyl)-éthylènediamine.

11. Mise en oeuvre des polymères hydrophiles réticulés sous forme de mousse selon les revendications 1 à 5 dans des produits hygiéniques que l'on emploie pour l'absorption des liquides biologiques, dans les produits de pansement pour couvrir les plaies, en tant que matériaux d'étanchéité, en tant qu'agent d'amendement du sol, en tant que produit de remplacement du sol et en tant que matériau d'emballage.